# EUROPEAN PATENT APPLICATION

(11) **EP 1 541 174 A1**
(43) Date of publication of application: **15.06.2005**
(21) Application number: 03795384.1
(22) Date of filing: 10.09.2003
(51) Int. Cl.: A61K 45/00, A61K 31/18, A61K 31/145

(54) **DRUG OR COSMETIC**

(30) Priority: 11.09.2002 JP 2002265884; 04.06.2003 JP 2003159975
(71) Applicant: Ishibashi, Michio, Sakai-shi, Osaka 590-0133 (JP)
(72) Inventor: Ishibashi, Michio, Sakai-shi, Osaka 590-0133 (JP)
(74) Representative: Teipel, Stephan, Dr.
(86) International application number: PCT/JP2003/011600
(87) International publication number: WO 2004/024185

(57) **Abstract**

The present invention provides pharmaceuticals or cosmetics, which prevent sclerotic lesions causing apoptosis, degeneration, fibrosis and atrophy, and repair and regenerate the lesions selectively, comprising a compound which preferentially increases regeneration-promoting macrophages and a method for screening the compound which preferentially increases regeneration-promoting macrophages. Pharmaceuticals or cosmetics of the present invention are useful for renal lesions, pancreatic lesions and skin lesions, because these induce regeneration-promoting immunocompetent cells lesion-selectively against organ tissue disorders and cause repair and regeneration thereof.

The present invention provides pharmaceuticals or cosmetics, which prevent sclerotic lesions causing apoptosis, degeneration, fibrosis and atrophy, and repair and regenerate the lesions selectively, comprising a compound which preferentially increases regeneration-promoting macrophages and a method for screening the compound which preferentially increases regeneration-promoting macrophages. Pharmaceuticals or cosmetics of the present invention are useful for renal lesions, pancreatic lesions and skin lesions, because these induce regeneration-promoting immunocompetent cells lesion-selectively against organ tissue disorders and cause repair and regeneration thereof.

## Description

### TECHNICAL FIELD

The present invention relates to a pharmaceutical and cosmetic that can prevent production and function of cytotoxic macrophages lesion-selectively causing a tissue disorder, inhibit cell processes of causing apoptosis, degeneration, fibrosis and atrophy, effect repair and regeneration of lesions, recover and save organ tissue functions, and prevent and/or treat a progressive disorder by inducing lesion-selective regeneration-promoting immunocompetent cells against an organ tissue disorder, or lesion-selective regulatory T lymphocytes and regeneration-promoting macrophages, and relates to a method for screening a compound that can be pharmaceuticals or cosmetics.

### BACKGROUND ART

Organ tissue disorders are caused by a variety of causes such as trauma; hypertension; hemodynamic circulatory insufficiency; ischemia-reperfusion injury; metabolic disorder; inflammatory induced by virus, cancer or bacterial infection; rejection; extraneous stress such as UV ray. As a radical therapy for the causes, an avoidance of an ischemic injury by a revascularization to an organ and the like is included. However, in general, cardiovascular and metabolic diseases are treated with an antihypertensive agent and a glucose metabolism-improving agent for avoiding cell disorders, and autoimmune diseases and organ transplant rejection are treated for the preservation of tissue by immunosuppressive therapy and symptomatic treatments. Additionally, steroids have various actions and are effective for refractory diseases, but an effective high dose thereof is difficult to be used for long time due to the occurrence of the side effect, and thus the inhibition of the progression of progressive organ tissue disorders cannot be achieved.

Once a cellular tissue has necrotized, progression therein is irreversible, and repair and regeneration thereof is impossible. Until now, for repair and regeneration of injured tissue cells, there is no alternative but to see hope in the tissue specific restorability and regeneration ability of an individual, and depending on the level of disorder, lesions lead to apoptosis, degeneration, fibrosis and atrophy, and finally to tissue organ dysfunction. A gene therapy that is intended to control processes of lesions causing apoptosis, degeneration, fibrosis and atrophy, and bring out the restorability and regeneration ability of an individual, and a study of regenerative medicine are at the experimental stage. There is still no pharmaceutical that induces repair and regeneration selectively against lesions and can be taken for a long time.

For the restorability and regeneration ability of an individual against lesions, it has been known that a leukocyte has some effect. For example, in an experimental study of cutaneous wound healing, it has been suggested that macrophages have some function on the repair and regeneration (Cell Tissue Res., vol.306: p.239-250, 2001). Also, for regeneration of organ, Manuela Ghielli et al. have suggested that a leukocyte plays some role in the recovery process from the acute renal failure, because leukocyte infiltration has been observed in the regeneration of renal tubule (Renal Failure, 1996, vol.18, p.355-375). However, its details have not yet been revealed.

The present inventor has shown that there are cytotoxic effector macrophages in progressive lesions after organ damage, which are produced in response to renal glomerular lesions and renal tubulointerstitial lesions respectively and consequently lead to the progression (deterioration) of each of the lesions selectively and that there are compounds selectively inhibiting the production of effector macrophages corresponding to each of the lesions (WO 01/72730).

Also, M Schaffer et al. have described that, in a rat model for evaluating skin repair from radiation damage, concentrations of interferon and TNF in tissue are excess (J. Surg. Res., 2002, vol.107: 93-100), and further, VK Rumella et al. have reviewed that, in the healing of chronic cutaneous wound, interferon inhibits regeneration of cutaneous epidermal cells, while TNF suppresses hyperplasia of collagen of cutaneous dermis (Plast. Reconstr. Surg., 2001, vol.108: 719-733). As described above, in skin, it has been suggested that there is a mechanism leading to the lesion-selective progressive lesions after cutaneous damage.

However, no compound inducing selective repair and regeneration against lesions is described and yet revealed.

### DISCLOSURE OF THE INVENTION

The object of the present invention is to provide a pharmaceutical for prevention and/or therapy of an organ tissue disorder that induces lesion-selective regeneration-promoting immunocompetent cells against the organ tissue disorder and leads to repair and regeneration lesion-selectively. That is, the object of the present invention is to provide a pharmaceutical for prevention and/or therapy of an organ tissue disorder, especially a progressive disorder that can prevent production and function of cytotoxic macrophages causing a lesion-selective tissue disorder, inhibit cell processes of causing apoptosis, degeneration, fibrosis and atrophy, effect repair and regeneration of lesions, and recover and save organ tissue functions by inducing lesion-selective regulatory T lymphocytes and regeneration-promoting macrophages.

Another object of the present invention is to provide a method for screening a compound that lesion-selectively induces regeneration-promoting immunocompetent cells inducing the immunoregulatory response of the repair and regeneration selectively on the lesion part, that is, regeneration-promoting macrophages or regulatory T lymphocytes.

Moreover, another object of the present invention is to provide a pharmaceutical that treats or prevents kidney diseases, pancreatic diseases, or skin diseases by using a compound having effects on renal glomerular lesions and tubulointerstitial lesions respectively, or a compound leading to repair and regeneration of tissues of renal glomerular lesions and tubulointerstitial lesions respectively alone or in combination.

Further, another object of the present invention is to provide a pharmaceutical or cosmetic having a preventive treatment effect for cutaneous epidermal lesions such as cutaneous wound healing, the prevention, improvement or treatment of an abnormal pigmentation and the prevention or improvement of skin aging by inducing reparative proliferation of epidermal cells and melanocytes against tissue disorders of cutaneous epidermis, and in addition, to provide a pharmaceutical or cosmetic having a preventive treatment effect for cutaneous dermal lesions such as cutaneous wound healing, the repair of skin turgor, the prevention, improvement or treatment of an abnormal pigmentation and the prevention or improvement of skin aging by inducing the reparative proliferation of collagen, elastic fiber and, glandular tissue and angiogenesis against the tissue disorders of cutaneous dermis.

The present inventors have studied for a chemokine receptor and an adhesion molecule of a macrophage based on the idea that a macrophage having a function of promoting regeneration of tissue in an organ disorder would exist. As a result, we unexpectedly have found that, when tissue is damaged, regeneration-promoting macrophages are induced to lead to repair and regeneration of the tissue in a selective mode to lesions. More specifically, the inventors of the present invention have found that CD11b⁺CD2⁺ macrophage is involved in repair and regeneration of renal glomerular lesions and CD11b⁻CD2⁺ macrophage is involved in repair and regeneration of renal tubulointerstitial lesions. Further, also studying for T lymphocytes, the present inventors have found that a regulatory CD2⁻CD4⁺ T lymphocyte is involved in repair and regeneration of tissue after organ damage.

Further, the present inventors have found that, in order to improve lesions of the whole kidney, an expedient control of immune response by selectively preventing cytotoxic macrophages consistent with both glomerular lesions and tubulointerstitial lesions by inducing regulatory T lymphocytes, and at the same time selectively inducing regeneration-promoting macrophages to local lesions is necessary. The inventors of the present invention have also found pharmaceuticals in which compounds having effects for glomerular lesions and tubulointerstitial lesions respectively are used in combination to treat kidney diseases.

As described above in the section of prior art, the present inventors have already found in WO 01/72730 that cytotoxic macrophages are produced in response to renal glomerular lesions and renal tubulointerstitial lesions respectively. By further studying this mechanism, the inventors of the present invention have found that the production of cytotoxic macrophages in renal glomerular lesions involves production and secretion of interferon and the production of cytotoxic macrophages in renal tubulointerstitial lesions involves production and secretion of TNF. As described previously, in the healing of chronic cutaneous wound, interferon inhibits regeneration of cutaneous epidermal cells, while TNF controls hyperplasia of collagen of cutaneous dermis (VK Rumella et al., Plast. Reconstr. Surg., 2001, vol.108: 719-733).

From the above-described findings and the description of VK Rumella et al., it is thought that progression of renal glomerular lesions and progression of epidermal lesions involve cytotoxic macrophages induced by the production and secretion of interferon, while progression of renal tubulointerstitial lesions and progression of dermal lesions involve cytotoxic macrophages induced by the production and secretion of TNF. It is also really thought that, if mechanisms of the progression of lesions are same, then mechanisms of the repair and regeneration of lesions are also same, in other words, in renal glomerular lesions and in epidermal lesions, same mechanisms of the repair and regeneration function, while in renal tubulointerstitial lesions and in dermal lesions, same mechanisms of the repair and regeneration function.

Considering these discussions, the present inventors have found from the results of the below-describing Examples that, in lesions in the kidney, by nature, regeneration-promoting macrophages corresponding to glomerular lesions and tubulointerstitial lesions respectively exist, and there is a mechanism preventing mechanisms leading to progressive lesions such as glomerular sclerosis, tubulointerstitial atrophy, and fibrosis.

The inventors of the present invention also have found from animal studies for an effect of a compound selectively inhibiting production of cytotoxic macrophages induced by glomerular lesions or tubulointerstitial lesions that the compound is a compound also having an effect capable of inducing lesion-selective regeneration-promoting macrophages corresponding to the lesions.

Further, as revealed by the administration studies of these compounds, the inventors of the present invention have found that, in order to improve lesions of the whole kidney, an expedient control of immune response by selectively preventing cytotoxic macrophages consistent with both glomerular lesions and tubulointerstitial lesions by inducing regulatory T lymphocytes, and at the same time selectively inducing regeneration-promoting macrophages to local lesions is necessary.

As described above, the present invention can provide a method for screening a compound inducing regulatory T lymphocytes and selective lesion-specific regeneration-promoting macrophages. The inventors of the present invention also have found that the compound obtained from the screening preferably prevents selectively cytotoxic macrophages at the same time for the improvement of lesions.

Considering a mechanism of cell disorder and a mechanism of repair and regeneration of tissue in progressive lesions, the inventors of the present invention unexpectedly have found that CD11b⁺CD2⁺ macrophage involved in repair and regeneration of renal glomerular lesions is also involved in repair and regeneration of cutaneous epidermal cells and CD11b⁻CD2⁺ macrophage involved in repair and regeneration of renal tubulointerstitial lesions is also involved in repair and regeneration of cutaneous dermal cells.

As described above, the inventors of the present invention have found that CD11b⁺CD2⁺ macrophage involved in repair and regeneration of renal glomerular lesions is also involved in repair and regeneration of cutaneous epidermal cells and CD11b⁻CD2⁺ macrophage involved in repair and regeneration of renal tubulointerstitial lesions is also involved in repair and regeneration of cutaneous dermal cells.

The present inventers have studied further to accomplish the present invention.

That is, the present invention relates to:
(1) a composition for prevention of sclerotic lesions causing apoptosis, degeneration, fibrosis and atrophy, and/or for repair and regeneration of the lesions, comprising a compound which preferentially increases regeneration-promoting macrophages;
(2) a pharmaceutical for prevention and/or therapy of renal glomerular lesions, lesions of pancreatic islets of Langerhans or epidermal lesions, comprising a compound which preferentially increases regeneration-promoting CD11b⁺CD2⁺ macrophages;
(3) the pharmaceutical described in (2), wherein the compound which preferentially increasing regeneration-promoting CD11b⁺CD2⁺ macrophages is at least one compound selected from the group consisting of (R)-1-naphthalen-2-ylethyl (R)-2-(4-fluorophenoxy)-5-oxotetrahydrofuran-2-carboxylate, 2-fluoro-5-oxotetrahydrofuran- 2-carboxylic acid benzyl ester, bacitracin A, viomycin, 6,7-dimethoxy-1-morpholinomethyl-isochromane, 1-diethylaminomethyl-5-butoxy-6-methoxy-1,2,3,4-tetrahydroisoquinoline, 1-(4-fluorophenylthio)-2-methylaminopropanone, 2-chloro-5-oxotetrahydrofuran-2-carboxylic acid benzyl ester and 1-(2-oxo-hemiglutaric acid) benzyl ester;
(4) a pharmaceutical for prevention and/or therapy of renal tubulointerstitial lesions, lesions of pancreatic exocrine or interstitial tissues, or dermal lesions, comprising a compound which promotes induction of regeneration-promoting CD11b⁻CD2⁺ macrophages; and
(5) the pharmaceutical described in (4), wherein the compound promoting induction of regeneration-promoting CD11b⁻CD2⁺ macrophages is at least one compound selected from the group consisting of (R)-1-naphthalen-2-ylethyl (S)-2-(4-fluorophenoxy)-5-oxotetrahydrofuran-2-carboxylate, 2-benzyl-5-oxo-2-tetrahydrofuran carboxylic acid benzyl ester, 1-chloro-3-oxo-1,3-dihydroisobenzofuran-1-carboxylic acid benzyl ester and 1-(2-oxo-hemiglutaric acid)ethyl ester.

Also, the present invention relates to:
(6) a pharmaceutical for prevention and/or therapy of kidney diseases, pancreatic diseases or skin diseases, comprising the compound described in (2) and the compound described in (4);
(7) a pharmaceutical for prevention and/or therapy of kidney diseases, pancreatic diseases or skin diseases, comprising a compound which promotes induction of regeneration-promoting CD11b⁺CD2⁺ macrophages and regeneration-promoting CD11b⁻CD2⁺ macrophages; and
(8) the pharmaceutical described in (7), wherein the compound promoting induction of regeneration-promoting CD11b⁺CD2⁺ macrophages and regeneration-promoting CD11b⁻CD2⁺ macrophages is at least one compound selected from the group consisting of 2-(4-chlorophenyl)thio-5-oxo-2-tetrahydrofuran carboxylic acid benzyl ester, 2-(4-fluorophenyl)oxy-5-oxo-2-tetrahydrofurancarboxylic acid ethyl ester, 2-(2,4-difluorophenyl)sulfonyl-5-oxo-2-tetrahydrofurancarboxylic acid benzyl ester, 2-phenoxy-5-oxo-2-tetrahydrofuran-carboxylic acid benzhydryl ester, 2-(4-fluorophenyl)thio-5-oxo-2-tetrahydrofurancarboxylic acid, 2-(4-methoxyphenyl)thio-5-oxo-2-tetrahydrofurancarboxylic acid, 2-(2,4-difluorophenyl)thio-5-oxo-2-tetrahydrofuran-carboxylic acid and 2-(4-fluorophenyl)sulfonyl-5-oxo-2-tetrahydrofurancarboxylic acid benzyl ester.

Further, the present invention relates to:
(9) a cosmetic comprising a compound which promotes induction of regeneration-promoting CD11b⁺CD2⁺ macrophages;
(10) the cosmetic described in (9), wherein the compound promoting induction of regeneration-promoting CD11b⁺CD2⁺ macrophages is at least one compound selected from the group consisting of (R)-1-naphthalen-2-ylethyl (R)-2-(4-fluorophenoxy)-5-oxotetrahydrofuran-2-carboxylate, 2-fluoro-5-oxotetrahydrofuran-2-carboxylic acid benzyl ester, bacitracin A, viomycin, 6,7-dimethoxy-1-morpholinomethyl-isochromane, 1-diethylaminomethyl-5-butoxy-6-methoxy-1,2,3,4-tetrahydroisoquinoline, 1-(4-fluorophenylthio)-2-methylaminopropanone, 2-chloro-5-oxotetrahydrofuran-2-carboxylic acid benzyl ester and 1-(2-oxo-hemiglutaric acid) benzyl ester;
(11) a cosmetic comprising a compound which promotes induction of regeneration-promoting CD11b⁻CD2⁺ macrophages;
(12) the cosmetic described in (11), wherein the compound promoting induction of regeneration-promoting CD11b⁻CD2⁺ macrophages is at least one compound selected from the group consisting of (R)-1-naphthalen-2-ylethyl (S)-2-(4-fluorophenoxy)-5-oxotetrahydrofuran-2-carboxylate, 2-benzyl-5-oxo-2-tetrahydrofuran carboxylic acid benzyl ester, 1-chloro-3-oxo-1,3-dihydroisobenzofuran-1-carboxylic acid benzyl ester and 1-(2-oxo-hemiglutaric acid) ethyl ester;
(13) a cosmetic comprising the compound described in (9) and the compound described in (11);
(14) a cosmetic comprising a compound which promotes induction of regeneration-promoting CD11b⁺CD2⁺ macrophages and regeneration-promoting CD11b⁻CD2⁺ macrophages; and
(15) the cosmetic described in (14), wherein the compound promoting induction of regeneration-promoting CD11b⁺CD2⁺ macrophages and regeneration-promoting CD11b⁻CD2⁺ macrophages is at least compound selected from the group consisting of 2-(4-chlorophenyl)thio-5-oxo-2-tetrahydrofuran carboxylic acid benzyl ester, 2-(4-fluorophenyl)oxy-5-oxo-2-tetrahydrofurancarboxylic acid ethyl ester, 2-(2,4-difluorophenyl)sulfonyl-5-oxo-2-tetrahydrofurancarboxylic acid benzyl ester, 2-phenoxy-5-oxo-2-tetrahydrofuran-carboxylic acid benzhydryl ester, 2-(4-fluorophenyl)thio-5-oxo-2-tetrahydrofurancarboxylic acid, 2-(4-methoxyphenyl)thio-5-oxo-2-tetrahydrofurancarboxylic acid, 2-(2,4-difluorophenyl)thio-5-oxo-2-tetrahydrofuran-carboxylic acid and 2-(4-fluorophenyl)sulfonyl-5-oxo-2-tetrahydrofurancarboxylic acid benzyl ester.

Moreover, the present invention relates to:
(16) a method for screening a compound which is able to prevent, mitigate or treat renal glomerular lesions, lesions of pancreatic islets of Langerhans or epidermal lesions, comprising measuring a promoting action of a test compound on the induction of regeneration-promoting CD11b⁺CD2⁺ macrophages and regulatory CD2⁻CD4⁺ T lymphocytes caused by contact of human peripheral blood mononuclear cells with lipopolysaccharide;
(17) the method for screening described in (16), comprising steps of inducing regeneration-promoting CD11b⁺CD2⁺ macrophages and regulatory CD2⁻CD4⁺ T lymphocytes by cultivating human peripheral blood mononuclear cells in RPMI 1640 medium in the presence of a test compound, lipopolysaccharide and human AB type serum, counting numbers of CD11b⁺CD2⁺ macrophages and CD2⁻CD4⁺ T lymphocytes, and determining increments of CD11b⁺CD2⁺ macrophages and CD2⁻CD4⁺ T lymphocyte in comparison to the case of the absence of the test compound;
(18) a method for screening a compound which is able to prevent, mitigate or treat renal glomerular lesions, lesions of pancreatic islets of Langerhans or epidermal lesions, comprising steps of (i) inducing cytotoxic macrophages by cultivating human peripheral blood mononuclear cells in RPMI 1640 medium in the presence of lipopolysaccharide and human AB type serum, at the same time, (ii) inducing unfractionated T lymphocytes by cultivating human peripheral blood unfractionated T lymphocytes with or without lipopolysaccharide in the presence of a test compound,(iii)counting a number of produced cytotoxic macrophages when the cytotoxic macrophages obtained in (i) are brought into contact with monolayered autologous erythrocytes in the presence of the unfractionated T lymphocytes obtained in (ii), and (iv) determining a decrement of a number of generated cytotoxic macrophages in comparison to the case of the absence of the test compound;
(19) a method for screening a compound which is able to prevent, mitigate or treat renal glomerular lesions, lesions of pancreatic islets of Langerhans or epidermal lesions, comprising steps of (i) inducing unfractionated T lymphocytes by cultivating human peripheral blood mononuclear cells in the presence of a test compound, human AB type serum and lipopolysaccharide, (ii) counting a number of regulatory CD2⁻CD4⁺ T lymphocytes in the induced unfractionated T lymphocytes, and (iii) determining an increment of the regulatory CD2⁻CD4⁺ T lymphocytes in comparison to the case of the absence of the test compound.

Furthermore, the present invention relates to:
(20) a method for screening a compound which is able to prevent, mitigate or treat renal tubulointerstitial lesions, lesions of pancreatic exocrine or interstitial tissues, or dermal lesions, comprising measuring a promoting action of a test compound on the induction of regeneration-promoting CD11b⁻CD2⁺ macrophages and/or regulatory CD2⁻CD4⁺ T lymphocytes caused by contact of human peripheral blood mononuclear cells with mitomycin-treated human peripheral blood mononuclear cells;
(21) the method for screening described in (20), comprising steps of inducing regeneration-promoting CD11b⁻CD2⁺ macrophages and/or regulatory CD2⁻CD4⁺ T lymphocytes by cultivating human peripheral blood mononuclear cells and mitomycin-treated human peripheral blood mononuclear cells in RPMI 1640 medium in the presence of a test compound and human AB type serum, counting numbers of CD11b⁻CD2⁺ macrophages and CD2⁻CD4⁺ T lymphocytes, and determining increments of CD11b⁻CD2⁺ macrophages and CD2⁻CD4⁺ T lymphocytes in comparison to the case of the absence of the test compound;
(22) a method for screening a compound which is able to prevent, mitigate or treat renal tubulointerstitial lesions, lesions of pancreatic exocrine or interstitial tissues, or dermal lesions, comprising steps of (i) inducing cytotoxic macrophages by cultivating human peripheral blood mononuclear cells and mitomycin-treated human peripheral blood mononuclear cells in RPMI 1640 medium in the presence of human AB type serum, at the same time, (ii) inducing unfractionated T lymphocytes by cultivating human peripheral blood unfractionated T lymphocytes with or without mitomycin-treated human peripheral blood mononuclear cells in the presence of a test compound,(iii)counting a number of produced cytotoxic macrophages when the cytotoxic macrophages obtained in (i) are brought into contact with monolayered autologous erythrocytes in the presence of the unfractionated T lymphocytes obtained in (ii), and (iv) determining a decrement of a number of produced cytotoxic macrophages in comparison to the case of the absence of the test compound; and
(23) a method for screening a compound which is able to prevent, mitigate or treat renal tubulointerstitial lesions, lesions of pancreatic exocrine or interstitial tissues , or dermal lesions, comprising steps of (i) inducing unfractionated T lymphocytes by cultivating mitomycin-treated human peripheral blood mononuclear cells and human peripheral blood mononuclear cells in the presence of a test compound and human AB type serum, (ii) counting a number of regulatory CD2⁻CD4⁺ T lymphocytes in the induced unfractionated T lymphocytes, and(iii) determining an increment of the regulatory CD2⁻CD4⁺ T lymphocytes in comparison to the case of the absence of the test compound.

Still further, the present invention relates to:
(24) a kit for screening a compound which is able to prevent, mitigate or treat renal glomerular lesions, lesions of pancreatic islets of Langerhans or epidermal lesions, comprising (a) human peripheral blood mononuclear cells, (b) lipopolysaccharide and optionally (c) human AB type serum and/or (d) RPMI 1640 medium; and
(25) a kit for screening a compound which is able to prevent, mitigate or treat renal tubulointerstitial lesions, lesions of pancreatic exocrine or interstitial tissues, or dermal lesions, comprising (a) human peripheral blood mononuclear cells, (b) mitomycin-treated human peripheral blood mononuclear cells and optionally (c) human AB type serum and/or (d) RPMI 1640 medium.

Further, the present invention relates to: a method for prevention and/or therapy of renal glomerular lesions, lesions of pancreatic islets of Langerhans and epidermal lesions and epidermal lesions, or renal tubulointerstitial lesions, lesions of pancreatic exocrine or interstitial tissues and dermal lesions, or kidney diseases, pancreatic diseases and skin diseases, comprising administering the pharmaceutical described in (2) to (8) to a patient; and the use of a compound increasing regeneration-promoting CD11b⁺CD2⁺ macrophages, a compound increasing regeneration-promoting CD11b⁻CD2⁺ macrophages and a compound promoting induction of regulatory CD2⁻CD4⁺ T lymphocytes for preparing the pharmaceutical described n (2) to (8).

As used herein, the term "increase (or promote) induction of regeneration-promoting CD11b⁺CD2⁺ macrophages" means mainly, but not limited to, a mechanism of inducing regulatory CD2⁻CD4⁺ T lymphocytes caused by contact of human peripheral blood mononuclear cells with lipopolysaccharide, preventing generation and function of cytotoxic macrophages to allow regeneration-promoting CD11b⁺CD2⁺ macrophages function dominantly in quantitative and qualitative manner, and means that regeneration-promoting CD11b⁺CD2⁺ macrophages function dominantly in quantitative and qualitative manner, including, for example, a case not via regulatory T lymphocytes. As used herein, "CD" is an abbreviation of "cluster of differentiation" , and "function dominantly in quantitative and qualitative manner" means a case that when macrophages increase quantitatively, functions of macrophages are dominant (the following "CD" and "function dominantly in quantitative and qualitative manner" have the same meanings). "CD11b⁺CD2⁺ macrophage" is referred to as a macrophage recognized by at least a monoclonal antibody against "CD11b⁺" and "CD2⁺" but does not exclude a CD recognized by other monoclonal antibody.

The term "promote induction of regeneration-promoting CD11b⁻CD2⁺ macrophages" means mainly, but not limited to, that regeneration-promoting CD11b⁻CD2⁺ macrophages caused by contact of human peripheral blood mononuclear cells with mitomycin-treated human peripheral blood mononuclear cells function dominantly in quantitative and qualitative manner, and means that regeneration-promoting CD11b⁻CD2⁺ macrophages function dominantly in quantitative and qualitative manner. As used herein, "CD11b⁻CD2⁺ macrophage" is referred to as a macrophage recognized by at least a monoclonal antibody against "CD11b⁻" and "CD2⁺" but does not exclude a CD recognized by other monoclonal antibody. For example, "CD11b⁻CD2⁺CD5⁺ macrophage" is especially preferable for "CD11b⁻CD2⁺ macrophage" in the present invention.

The term "regeneration" means repair and regeneration of damage, including the repair of damage.

The term "regulatory" in "regulatory CD2⁻CD4⁺ T lymphocyte" means it controls cytotoxic macrophages and regeneration-promoting macrophages. Also, "CD2⁻CD4⁺ T lymphocyte" means a T lymphocyte recognized by at least a monoclonal antibody against "CD2⁻" and "CD4⁺" but does not exclude a CD recognized by other monoclonal antibody.

A compound which increases induction of regeneration-promoting CD11b⁺CD2⁺ macrophages used in the present invention is any compound having any mechanism as long as it increases or promotes induction of regeneration-promoting CD11b⁺CD2⁺ macrophages, including, for example, a compound inducing regulatory CD2⁻CD4⁺ T lymphocytes, preventing the generation and function of cytotoxic macrophages and promoting induction of regeneration-promoting CD11b⁺CD2⁺ macrophages, a compound promoting induction of regeneration-promoting CD11b⁺CD2⁺ macrophages not via regulatory T-lymphocytes, a compound promoting induction of regeneration-promoting CD11b⁺CD2⁺ macrophages caused by contact of human peripheral blood mononuclear cells and lipopolysaccharide (e.g., LPS), and the like.

A compound which promotes induction of regulatory CD2⁻CD4⁺ T lymphocytes is any compound having any mechanism as long as it induces regulatory CD2⁻CD4⁺ T lymphocytes and prevents generation and function of cytotoxic macrophages, including, for example, a compound promoting induction of regeneration-promoting CD11b⁺CD2⁺ macrophages caused by contact of human peripheral blood mononuclear cells and lipopolysaccharide (e.g., LPS) and the like.

A compound which promotes induction of regeneration-promoting CD11b⁻CD2⁺ macrophages used in the present invention is any compound having any mechanism as long as it promotes induction of regeneration-promoting CD11b⁻CD2⁺ macrophages, including, for example, a compound inducing regulatory CD2⁻CD4⁺ T lymphocytes, preventing the generation and function of cytotoxic macrophages and promoting induction of regeneration-promoting CD11b⁻CD2⁺ macrophages, a compound promoting induction of regeneration-promoting CD11b⁻CD2⁺ macrophages not via regulatory T-lymphocytes, a compound promoting induction of regeneration-promoting CD11b⁻CD2⁺ macrophages caused by contact of human peripheral blood mononuclear cells and mitomycin-treated human allogeneic peripheral blood mononuclear cells and the like.

Examples of the compound which promotes induction of regeneration-promoting CD11b⁺CD2⁺ macrophages used in the present invention (hereinafter, referred to as induction-promoting compound for regeneration-promoting CD11b⁺CD2⁺ macrophages.) include: (R)-1-naphthalen-2-ylethyl (R)-2-(4-fluorophenoxy)-5-oxotetrahydrofuran-2-carboxylate (compound (II-1)), described in WO 01/72730, represented by the following formula (II-1); 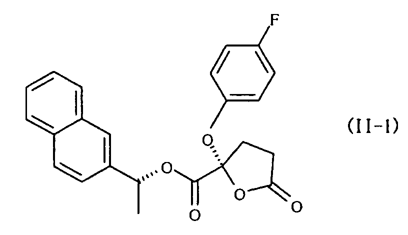 2-fluoro-5-oxotetrahydrofuran-2-carboxylic acid benzyl ester (compound (II-2)), represented by the following formula (II-2); 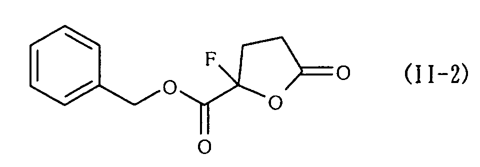 a compound (compound (II-3)), described in J. Antibiotics, Vol. 37, p. 1546 (1984), represented by the following formula (II-3); 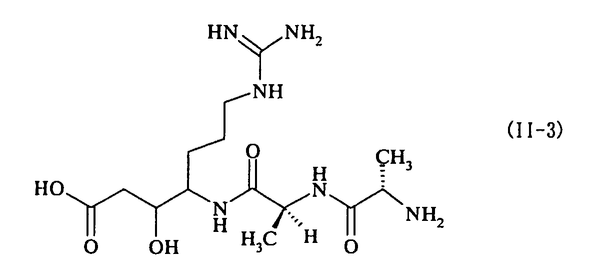 a compound (compound (II-4)), described in RO 88594, for example, represented by the following formula (II-4); 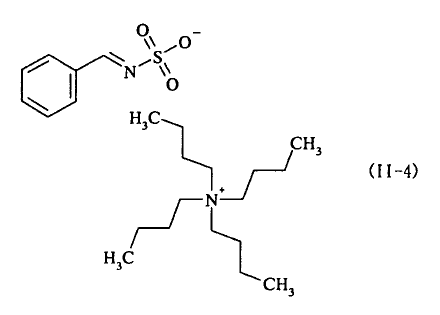 bacitracin A (compound (II-5)), described in Science, Vol.102, p.376 (1945), represented by the following formula (II-5); 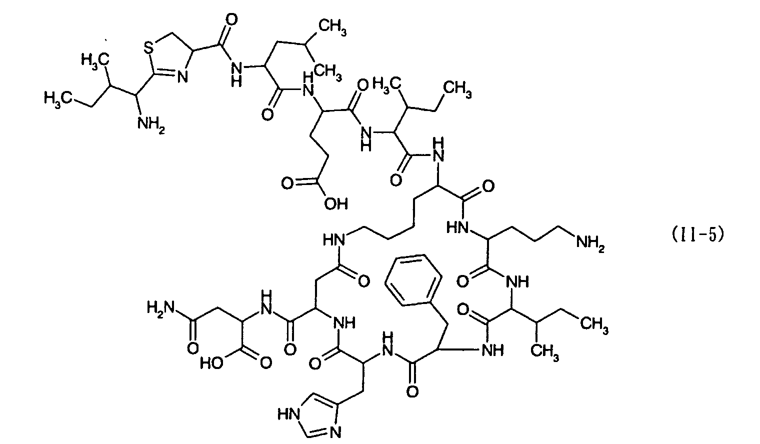 viomycin (compound (II-6)), represented by the following formula (II-6); 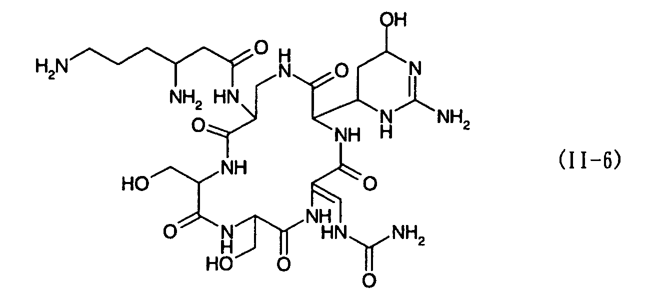 6,7-dimethoxy-1-morpholinomethyl-isochromane (compound (II-7)), described in JP-A-83846/1977, for example, represented by the following formula (II-7); 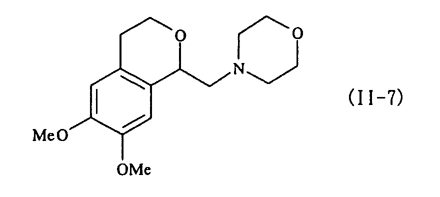 (wherein, Me represents a methyl group.)
1-diethylaminomethyl-5-butoxy-6-methoxy-1,2,3,4-tetrahydroi soquinoline (compound (II-8)), described in JP-A-139072/1977, for example, represented by the following formula (II-8); 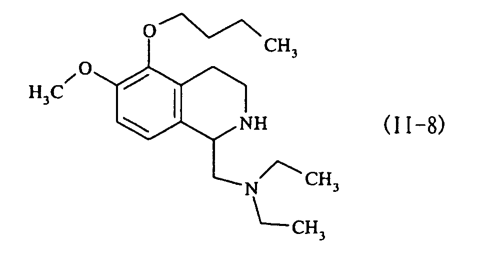 1-(4-fluorophenylthio)-2-methylaminopropanone (compound (II-9)), described in EP 53015, for example, represented by the following formula (II-9); 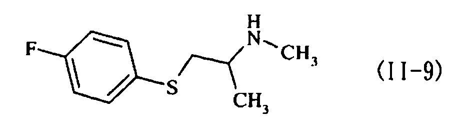 2-chloro-5-oxotetrahydrofuran-2-carboxylic acid benzyl ester (compound (II-10)), described in WO 01/72730, represented by the following formula (II-10); 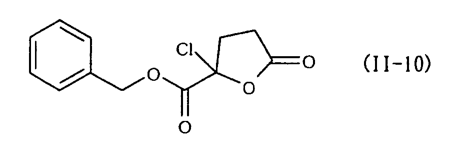 and a compound represented by the following formula (II-11); 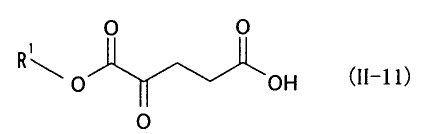 (wherein R¹ represents C₆₋₁₄ aryl group or C₇₋₁₆ aralkyl group.).

In the compound represented by the formula (II-11), the C₆₋₁₄ aryl group as the substituent R¹ includes, for example, phenyl group, biphenyl group Indenyl group and naphthyl group or a partially saturated group thereof such as 2,3-dihydroindenyl group and 1,2,3,4-tetrahydronaphthyl group. The C₇₋₁₆ aralkyl group as the substituent R¹ includes, for example, benzyl group, phenethyl group, phenylpropyl group, phenylbutyl group and phenylhexyl group. As the substituent R¹, benzyl group Is preferred.

These C₆₋₁₄ aryl group and C₇₋₁₆ aralkyl group may be substituted with a substituent within the chemically acceptable range. Examples of the substituent include: halogen, preferably such as fluorine, chlorine and bromine; oxo group; alkanoyl group, preferably C₁₋₈ alkanoyl such as acetyl and propionyl; alkanoyloxy group, preferably C₁₋₈ alkanoyloxy such as acetyloxy and propionyloxy; alkanoylamino group, preferably C₁₋₈ alkanoylamino such as acetylamino and propionylamino; carboxy group; alkoxycarbonyl group, preferably C₂₋₈ alkoxycarbonyl such as methoxycarbonyl and ethoxycarbonyl; haloalkylcarbonyl group, preferably C₂₋₈; alkoxy group, preferably C₁₋₈; haloalkoxy group, preferably C₁₋₈ haloalkoxy such as chloromethoxy and bromoethoxy; alkyl group, preferably C₁₋₂₀ alkyl such as methyl and ethyl; amino group; alkylamino group, preferably C₁₋₈; dialkylamino group, preferably C₂₋₁₆; cyclic amino group such as morpholino and piperidino; alkylaminocarbonyl group, preferably C₂₋₈; carbamoyl group; hydroxyl group; nitro group; cyano group; mercapto group; alkylthio group, preferably C₁₋₈; alkylsulfonyloxy group, preferably C₁₋₈; alkylsulfonylamino group, preferably C₁₋₈; and phenyl group.

Among the compounds represented by the formula II-11, especially preferred is 1-(2-oxo-hemiglutaric acid) benzyl ester (compound (II-11a)), described in WO 01/72730, represented by the following formula (II-11a); 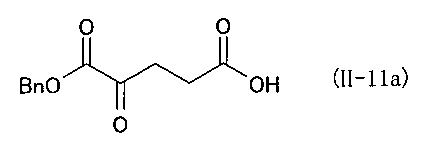 (wherein Bn represents a benzyl group.)
or a pharmacologically acceptable salt thereof.

Among the induction-promoting compounds for regeneration-promoting CD11b⁺CD2⁺ macrophages used in the present invention, (R)-1-naphthalen-2-ylethyl (R)-2-(4-fluorophenoxy)-5-oxotetrahydrofuran-2-carboxylate and 2-fluoro-5-oxotetrahydrofuran-2-carboxylic acid benzyl ester are especially preferred.

Examples of the compound which promotes induction of regeneration-promoting CD11b⁻CD2⁺ macrophages used in the present invention (hereinafter, referred to as a induction-promoting compound for regeneration-promoting CD11b⁻CD2⁺ macrophages) include: (R)-1-naphthalen-2-ylethyl (S)-2-(4-fluorophenoxy)-5-oxotetrahydrofuran-2-carboxylate (compound (I-1)), described in WO 01/72730, represented by the following formula (I-1); 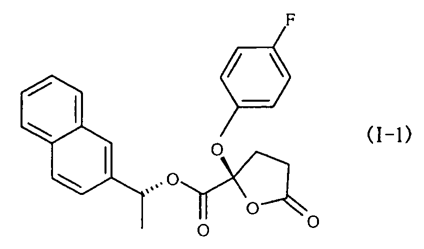 2-benzyl-5-oxo-2-tetrahydrofuran carboxylic acid benzyl ester (compound (I-2)), represented by the following formula (I-2); 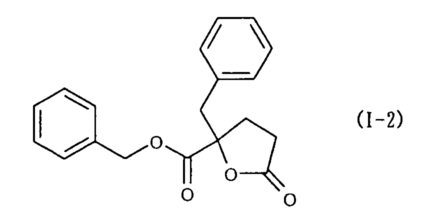 1-chloro-3-oxo-1,3-dihydroisobenzofuran-1-carboxylic acid benzyl ester (compound (I-3)), represented by the following formula (I-3); 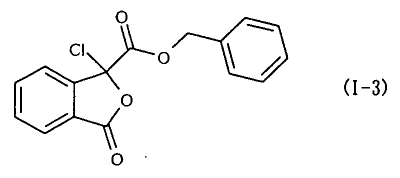 and a compound represented by the following formula (I-4); 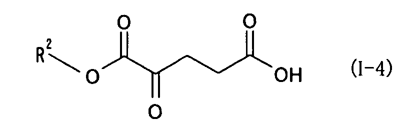 (wherein R² represents C₁₋₆ lower alkyl group or C₂₋₆ lower alkenyl group.).

In the compound represented by the formula (I-4), the substituent R² is C₁₋₆ lower alkyl group such as methyl group, ethyl group, n-propyl group Isopropyl group, n-butyl group Isobutyl group, tert-butyl group, n-pentyl group Isopentyl group, tert-pentyl group, n-hexyl group, 1,1-dimethylpropyl group and 3-methyl-3-butenyl group, or C₂₋₆ lower alkenyl group such as vinyl group, allyl group, 1-propenyl group Isopropenyl group, 2-butenyl group, 1,3-butadienyl group and 2-pentenyl group. Among them, preferred are ethyl group and butyl group, and especially preferred is ethyl group. These C₁₋₆ lower alkyl group and C₂₋₆ lower alkenyl group may be substituted with a substituent within the chemically acceptable range. Examples of the substituent include halogen, hydroxyl group, amino group, nitro group, cyano group, mercapto group, carbamoyl group, alkanoyl group, alkanoyloxy group and alkanoylamino group.

Among the compounds represented by the formula I-4, especially preferred is 1-(2-oxo-hemiglutaric acid)ethyl ester (compound (I-4a)), represented by the following formula (I-4a); 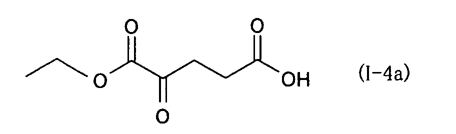 or a pharmacologically acceptable salt thereof.

Among the induction-promoting compounds for regeneration-promoting CD11b⁻CD2⁺ macrophages used in the present invention, (R)-1-naphthalen-2-ylethyl (S)-2-(4-fluorophenoxy)-5-oxotetrahydrofuran-2-carboxylate and 1-chloro-3-oxo-1,3-dihydroisobenzofuran-1-carboxylic acid benzyl ester are especially preferred.

The induction-promoting compound for regeneration-promoting CD11b⁺CD2⁺ macrophages and the induction-promoting compound for regeneration-promoting CD11b⁻CD2⁺ macrophages may be used in combination.

In the present invention, a compound which promotes induction of both macrophages described above may also be used.

Examples of the compound which promotes induction of both macrophages described above used in the present invention, or the compound which promotes induction of both regeneration-promoting CD11b⁺CD2⁺ macrophages and regeneration-promoting CD11b⁻CD2⁺ macrophages include a compound, described in JP-A-34976/1989 and JP-A-338331/1992, represented by the formula (III); 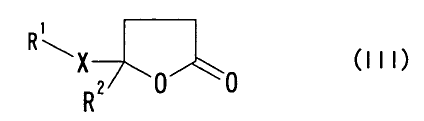 (wherein R¹ represents a phenyl group which may be substituted with phenyl group having 1 to 3 groups selected from halogen (fluorine, chlorine, bromine, and iodine) and C₁₋₃ alkoxy; R² represents carboxy group which may be esterified with, for example, alkyl or aralkyl; X represents oxygen or sulfur which may be oxidized.). Specific examples include: 2-(4-chlorophenyl)thio-5-oxo-2-tetrahydrofurancarboxylic acid benzyl ester (compound (III-1)), represented by the following formula (III-1); 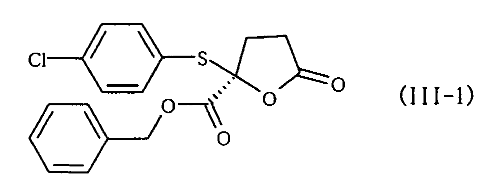 2-(4-fluorophenyl)oxy-5-oxo-2-tetrahydrofuran carboxylic acidethyl ester (compound (III-2)), represented by the following formula (III-2); 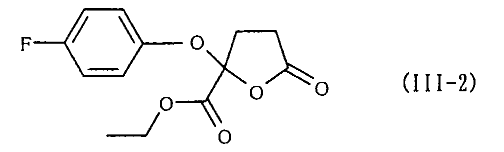 2-(2,4-difluorophenyl)sulfonyl-5-oxo-2-tetrahydrofuran-carboxylic acid benzyl ester; 2-phenoxy-5-oxo-2-tetrahydrofuran carboxylic acid benzhydryl ester; 2-(4-fluorophenyl)thio-5-oxo-2-tetrahydrofurancarboxylic acid; 2-(4-methoxyphenyl)thio-5-oxo-2-tetrahydrofurancarboxylic acid; 2-(2,4-difluorophenyl)thio-5-oxo-2-tetrahydrofuran-carboxylic acid; and 2-(4-fluorophenyl)sulfonyl-5-oxo-2-tetrahydrofurancarboxylic acid benzyl ester and the like.

Among the compounds which promote induction of regeneration-promoting CD11b⁺CD2⁺ macrophages and regeneration-promoting CD11b⁻CD2⁺ macrophages used in the present invention, 2-(4-chlorophenyl)thio-5-oxo-2-tetrahydrofurancarboxylic acid benzyl ester and 2-(4-fluorophenyl)oxy-5-oxo-2-tetrahydrofurancarboxylic acid ethyl ester are especially preferred.

The compound may be a derivative thereof. The derivative can be any compound, including a compound in which a hydrogen atom, especially a hydrogen atom of CH₃, CH₂, CH, NH, NH₂, COOH or OH is substituted with a usual substituent used in the art. Examples of the substituent include: halogen, preferably such as fluorine, chlorine and bromine; oxo group; alkyl group, preferably C₁₋₈; alkanoyl group, preferably C₁₋₈; alkanoyloxy group preferably C₁₋₈; alkanoylamino group, preferably C₁₋₈; carboxy group; alkoxycarbonyl group, preferably C₂₋₈; haloalkylcarbonyl group, preferably C₂₋₈; alkoxy group, preferably C₁₋₈; haloalkoxy group, preferably C₁₋₈; amino group; alkylamino group, preferably C₁₋₈; dialkylamino group, preferably C₂₋₁₆; cyclic amino group; alkylaminocarbonyl group, preferably C₂₋₈; carbamoyl group; hydroxyl group; nitro group; cyano group; mercapto group; alkylthio group, preferably C₁₋₈; alkylsulfonyloxy group, preferably C₁₋₈; alkylsulfonylamino group, preferably C₁₋₈; phenyl group; and benzyl group. Specific examples of these groups may be the same as of those described above.

Examples of the derivative also include a reduced form and an oxidized form of the above-described compound. Further, examples of the derivative include a compound in which a substituent is substituted with another substituent. For example, such derivatives include: a compound in which a halogen atom is substituted with another halogen atom; a compound in which a hydroxyl group Is substituted with a mercapto group; a compound in which an ether linkage is substituted with a thioether linkage; a compound in which a thioether linkage is substituted with an ether linkage; a compound in which an oxygen atom of heterocyclic group Is substituted with a nitrogen atom; and a compound in which a nitrogen atom of heterocyclic group Is substituted with an oxygen atom.

Above-described compounds are known compounds and can be produced easily according to the known method such as those described in WO 01/72730, JP-A-83846/1977, JP-A-139072/1977, EP 53015, J. Antibiotics, Vol.37, p.1546 (1984), RO 88594, Science, vol.102, p.376 (1945), USP 2633445, JP-A-34986/1989, JP-A-338331/1992. Derivatives of these compounds can also be produced easily from the compounds according to the method known in the art.

When the compound has an acidic or basic nature, a salt thereof may be used. Examples of the salt thereof include a physiologically acceptable salt with acids such as inorganic acid and organic acid, bases such as inorganic base and organic base, and amino acids such as acidic amino acid and basic amino acid. More specifically, when a compound has a basic nature, examples of the salt thereof include: an inorganic acid salt such as a salt with hydrochloric acid, phosphoric acid, hydrobromic acid and sulfuric acid; and an organic acid salt such as a salt with acetic acid, formic acid, propionic acid, fumaric acid, maleic acid, succinic acid, tartaric acid, citric acid, malic acid, oxalic acid, benzoic acid, methanesulfonic acid and benzenesulfonic acid. In this case, an acidic amino acid salt such as a salt with aspartic acid and glutamic acid may be formed. When a compound shows acidic activity, examples of the salt thereof include: inorganic base salt such as a salt with alkaline metal (e.g., sodium and potassium) and alkaline earth metal (e.g., magnesium and calcium); and organic base salt such as a salt with trimethylamine, triethylamine, pyridine, picoline, ethanolamine, diethanolamine, triethanolamine, dicyclohexylamine, N,N'-dibenzylethylenediamine. In this case, a basic amino acid salt such as a salt with arginine, lysine, and ornithine may be formed.

Alternatively, the compound may be a prodrug.

A compound used in the present invention is not limited to the above-described compound or a derivative thereof, and includes all compounds which can be obtained according to the following method of screening.

Specific examples of the renal glomerular lesion to which the induction-promoting compound for regeneration-promoting CD11b⁺CD2⁺ macrophages of the present invention such as the above-described compounds (II-1) to (II-11) and the compound (II-11a) can be applied include glomerulonephritis, nephritic syndrome, viral infectious disease, neoplastic disease, hematological disorder, immune system dysfunction, glomerular disorder in diabetes and the like.

Specific examples of the lesion of pancreatic islets of Langerhans to which the induction-promoting compound for regeneration-promoting CD11b⁺CD2⁺ macrophages of the present invention such as the above-described compounds (II-1) to (II-11) and the compound (II-11a) can be applied include diabetes and the like.

The epidermal lesion to which the induction-promoting compound for regeneration-promoting CD11b⁺CD2⁺ macrophages of the present invention such as the above-described compounds (II-1) to (II-11) and the compound (II-11a) can be applied includes lesions mainly accompanying cutaneous epidermal diseases, including contact dermatitis, palm eczema, diaper dermatitis, atopic dermatitis, senile pompholyx, Darier's disease, acanthosis nigricans, eczema, dermatitis herpetiformis and the like.

Specific examples of the renal tubulointerstitial lesion to which the induction-promoting compound for regeneration-promoting CD11b⁻CD2⁺ macrophages of the present invention such as the above-described compounds (I-1) to (I-4) and the compound (I-4a) can be applied include tubulointerstitial nephritis, hydronephrosis by obstructive uropathy, hydronephrosis, reflux nephropathy, viral infectious disease, neoplastic disease, hematological disorder and immune system dysfunction, and renal tubulointerstitial disorder in metabolism and the like.

Specific examples of the lesion of pancreatic exocrine or interstitial tissues to which the induction-promoting compound for regeneration-promoting GD11b⁻CD2⁺ macrophages of the present invention such as the above-described compounds (I-1) to (I-4) and the compound (I-4a) can be applied include pancreatitis and the like.

The dermal lesion to which the induction-promoting compound for regeneration-promoting CD11b⁻CD2⁺ macrophages of the present invention such as the above-described compounds (I-1) to (I-4) and the compound (I-4a) can be applied includes lesions mainly accompanying cutaneous dermal diseases, including folliculitis, urticaria pigmentosum, keloid, fibromatosis, anetoderma, striae atrophicae, senile atrophy, Kyrle disease, perforating folliculitis, sarcoidosis, foreign body granuloma, hyperhidrosis, sudamina, acne vulgaris of fingers, alopecia areata. The dermal lesion includes a case of accompanying lesions of not only skin appendage such as sweat gland and papilla pili, collagen and elastic fibers but also adipose tissue.

The compound which promotes induction of regeneration-promoting CD11b⁺CD2⁺ macrophages and regeneration-promoting CD11b⁻CD2⁺ macrophages used in the present invention such as a compound represented by formula (III) can be advantageously used in either of the above-described renal glomerular lesions and the above-described renal tubulointerstitial lesions, and in the case of having both lesions, or the overall kidney diseases including both.

The compound which promotes induction of regeneration-promoting CD11b⁺CD2⁺ macrophages and regeneration-promoting CD11b⁻CD2⁺ macrophages used in the present invention, such as a compound represented by formula (III), can be advantageously used in either of the above-described lesions of pancreatic islets of Langerhans and the above-described lesions of pancreatic exocrine or interstitial tissues, and in the case of having both lesions, or the overall pancreatic diseases including both.

Moreover, the compound which promotes induction of regeneration-promoting CD11b⁺CD2⁺ macrophages and regeneration-promoting CD11b⁻CD2⁺ macrophages used in the present invention, such as a compound represented by formula (III), can be advantageously used in either of the above-described cutaneous epidermal lesions and dermal lesions, and in the case of having both lesions , or the overall skin diseases including both such as lupus erythematosus, psoriasis, pigmentosa, bullous disease.

The regeneration-promoting CD11b+CD2+ macrophages and the regeneration-promoting CD11b⁻CD2⁺ macrophages are involved in renal glomerular lesions and renal tubulointerstitial lesions, or lesions of pancreatic islets of Langerhans and lesions of pancreatic exocrine or interstitial tissues, or epidermal lesions and dermal lesions, and each of the above-described compound promoting induction of these macrophages can be used, alone thereof or by blending with a pharmaceutically acceptable additive, to prepare pharmaceuticals for prevention and/or therapy (hereinafter, also referred to as a preventive and therapeutic agent) and pharmaceutical compositions of the renal glomerular lesions and renal tubulointerstitial lesions, or the lesions of pancreatic islets of Langerhans and lesions of pancreatic exocrine or interstitial tissues, or the epidermal lesions and dermal lesions. Alternatively, with reference to effects on the epidermal lesions and dermal lesions, by using each of the above-described compound promoting induction of the above-described macrophages, cosmetics can be prepared.

The pharmaceutical according to the present invention can be of any known dosage form, including those having such dosage form as tablet coated with sugar or film as needed, capsule, elixir, microcapsule and it can be administered orally. Those having such dosage form as sterile solution in water or the other pharmaceutically acceptable liquid or injectable such as suspension may be administered parenterally. The dosage form can be formed by the known method per se.

When the pharmaceutical according to the present invention is made to an internal medicine, it may contain an additive used in the related art, such as a flavor, an excipient, a vehicle, an antiseptic, a stabilizer and a binder. When made to tablets or capsules, examples of an additive which may be mixed include: a binder such as gelatin, corn starch, tragacanth and gum arabic; an excipient such as crystalline cellulose; a swelling agent such as corn starch, gelatin and alginic acid; a lubricant such as magnesium stearate; a sweetener such as sucrose, lactose and saccharine; a flavor such as peppermint, oil derived from Gaultheria adenothrix and cherry. When the dosage form is a capsule, a liquid carrier such as fat/oil may further be contained in addition to the above additives.

When the pharmaceutical according to the present invention is formulated into a liquid medicine such as injectable solution, there may be used, as an aqueous medium, for example, distilled water for injection, physiological saline and other isotonic solution containing glucose and other adjuvant such as D- sorbitol, D-mannitol and sodium chloride. In this case, an appropriate solubilizer such as alcohol(e.g., ethanol), polyalcohol(e.g., propylene glycol and polyethylene glycol) and nonionic surfactant (e.g., Polysorbate 80 (Trade mark) and HCO-50) may be added. As an oily liquid, sesame oil and soy oil and the like may be used. A solubilizer such as benzyl benzoate and benzyl alcohol may be also used together. Further, buffer (e.g., phosphate buffer and sodium acetate buffer), a soothing agent (e.g., benzalkonium chloride and procaine hydrochloride), a stabilizer (e. g. , human serum albumin and polyethylene glycol), a preservative (e.g., benzyl alcohol and phenol) and an antioxidant may be mixed optionally. A liquid medicine such as injectable solution prepared is usually filled in appropriate ampoules and vials. Alternatively, a liquid medicine may be prepared by dissolving just before use.

When the pharmaceutical according to the present invention is made to an external medicine, its dosage form may be of, for example, paste, ointment, cream, gel, gel-like cream, lotion, emulsion, suspension, compress, plaster, liniment, aerosol, syrup, intraoral agent, eye drop or nasal drop. Among them, as a preventive and therapeutic agent for skin diseases of the present invention, ointment, cream, gel, gel- like cream, lotion and emulsion are preferable.

When the pharmaceutical according to the present invention is an ointment, the preventive and therapeutic agent for skin diseases of the present invention can be prepared by blending a base such as a fat/oil base (e. g. , petrolatum, liquid paraffin, silicon and vegetable oil), an emulsion base (e.g., hydrophilic petrolatum and purified lanolin) and a water-soluble base such as macrogol with the compound of the present invention or a pharmacologically acceptable salt thereof and a pharmaceutical additive as needed such as an emulsifier (e.g., anionic and non-ionic surfactants) and a preservative (e.g., paraoxybenzoic acid esters).

When the pharmaceutical according to the present invention is a gel, the preventive and therapeutic agent for skin diseases of the present invention can be prepared by blending a base, which is obtained by adding a gelling agent (e.g., carboxyvinyl polymer, hydroxyethyl cellulose, hydroxypropyl cellulose, methylcellulose, ethylcellulose, carboxymethyl cellulose and propylene glycol alginate) to water, with the compound of the present invention or a pharmacologically acceptable salt thereof and a pharmaceutical additive as needed such as a lower alcohol, a neutralizing agent, a surfactant and an absorption promoter.

When the pharmaceutical according to the present invention is a cream, a base containing such as higher fatty acid ester (e.g., myristic acid ester, palmitic acid ester, diethyl sebacate, hexyl laurate and cetyl isooctanoate), lower alcohol (e.g., ethanol and isopropanol), hydrocarbon (e.g., liquid paraffin and squalane), polyhydric alcohol (e.g., propylene glycol and 1,3-butylene glycol) and higher alcohol (e.g., 2-hexyldecanol, cetanol, 2-octyl-dodecanol) may be blended with a pharmaceutical additive as needed such as an emulsifier, an antiseptic, an absorption promoter and a rash inhibitor.

To make a gel-like cream having a property intermediate between cream and gel, it may be prepared by addition of a gelling agent and a neutralizing agent.

When the pharmaceutical according to the present invention is an external liquid medicine, it may be blended with a solvent and a pharmaceutical additive as needed such as a buffer, a stabilizer, an antiseptic, a pH adjuster, a solvent, a solubilizer, a flavoring agent, a gelling agent, a taste masking agent and a refrigerant. Examples of the solvent include glycerol, propylene glycol, ethanol, isopropanol, butylene glycol, water, sorbitol, mannitol, xylitol, glucose, epsilon amino caproic acid, glycine, glutamic salt, sodium hyaluronate, polyethylene glycol, carboxyvinyl polymer, higher alcohol (e. g. , cetanol and stearyl alcohol), fatty acid ester (e.g., medium chain fatty acid ester and isopropyl myristate), higher fatty acid (e.g., stearic acid), squalane, liquid paraffin, white petrolatum and purified lanolin.

The external liquid medicine include a liquid formulation forexternalusesuchaswashing, injection, packing, inhalation, spraying, suppository, spreading, medicated bath, bed bath, disinfection, eye drop, eye wash, ear drop and nasal drop.

By using the external liquid medicine of the present invention together with a usual propellant, an aerosol can be prepared. Examples of the propellant include dimethyl ether, liquefied petroleum gas, N₂ gas, nitrous oxide gas, CO₂ gas and alternatives for chlorofluorocarbon gas, which are generally used in aerosol. Compressed air may be used instead of a propellant. Alternatively, a mixture thereof may be used.

The pharmaceuticals according to the present invention can contain other pharmaceutically active ingredient so far as it does not interfere the actions and effects of the present invention.

A pharmaceutical containing the induction-promoting compound for regeneration-promoting CD11b⁺CD2⁺ macrophages of the present invention is useful, as a preventive and therapeutic agent for renal glomerular lesions, such as glomerulonephritis, nephritic syndrome, viral infectious disease, neoplastic disease, hematological disorder and immune system dysfunction, and glomerular disorder in diabetes and the like.

A pharmaceutical containing the induction-promoting compound for regeneration-promoting CD11b⁻CD2⁺ macrophages of the present invention is useful, as a preventive and therapeutic agent for renal tubulointerstitial lesions, for such as tubulointerstitial nephritis, hydronephrosis by obstructive uropathy, hydronephrosis, reflux nephropathy, infectious disease, neoplastic disease, hematological disorder and immune system dysfunction, and renal tubulointerstitial disorder in metabolism and the like.

A pharmaceutical containing the compound which promotes induction of regeneration-promoting CD11b⁺CD2⁺ macrophages and regeneration-promoting CD11b⁻CD2⁺ macrophages of the present invention is useful as a preventive and therapeutic agent for kidney diseases because it is expected to have effects on either the renal glomerular lesions and the renal tubulointerstitial lesions.

When using the pharmaceutical of the present invention for kidney diseases, it is especially preferable to use concomitantly a pharmaceutical containing a compound which promotes induction of regeneration-promoting CD11b⁺CD2⁺ macrophages and regeneration-promoting CD11b⁻CD2⁺ macrophages, or to use concomitantly a preventive and therapeutic agent for renal glomerular lesions and a preventive and therapeutic agent for renal tubulointerstitial lesions.

A pharmaceutical containing the induction-promoting compound for regeneration-promoting CD11b⁺CD2⁺ macrophages of the present invention is useful as a preventive and therapeutic agent for lesions of pancreatic islets of Langerhans in treating diabetes and the like.

A pharmaceutical containing the induction-promoting compound for regeneration-promoting CD11b⁻CD2⁺ macrophages of the present invention is useful, as a preventive and therapeutic agent for lesions of pancreatic exocrine or interstitial tissues, in treating pancreatitis and the like.

A pharmaceutical containing the compound which promotes induction of regeneration-promoting CD11b⁺CD2⁺ macrophages and regeneration-promoting CD11b⁻CD2⁺ macrophages of the present invention is useful as a preventive and therapeutic agent for pancreatic diseases, because it is expected to have effects on either the lesions of pancreatic islets of Langerhans or the lesions of pancreatic exocrine or interstitial tissues.

Moreover, in the present invention, the preventive and therapeutic agent for lesions of pancreatic islets of Langerhans according to the present invention and the preventive and therapeutic agent for lesions of pancreatic exocrine or interstitial tissues according to the present invention may be concomitantly used.

When using the pharmaceutical of the present invention on pancreatic diseases, it is especially preferable to use a pharmaceutical containing a compound which promotes induction of regeneration-promoting CD11b⁺CD2⁺ macrophages and regeneration-promoting CD11b⁻CD2⁺ macrophages, or to use concomitantly a preventive and therapeutic agent lesions of pancreatic islets of Langerhans and a preventive and therapeutic agent for lesions of pancreatic exocrine or interstitial tissues together.

A pharmaceutical containing the induction-promoting compound for regeneration-promoting CD11b⁺CD2⁺ macrophages of the present invention is useful as a preventive and therapeutic agent for epidermal lesions in promoting healing of cut, cracked skin such as by burn, chapped skin, irritated skin, trauma, contact dermatitis, hand eczema, diaper dermatitis, atopic dermatitis, senile pompholyx, Darier's disease, acanthosis nigricans, eczema, dermatitis herpetiformis, molluscum contagiosum and the like, and in preventing and improving of skin aging such as wrinkle, darkness, pigmentation and skin roughness, because it has a promoting effect on a proliferation of melanocytes or epidermal cells, especially epidermal keratinocytes or a normalizing effect on epidermal cell function.

A pharmaceutical containing the induction-promoting compound for regeneration-promoting CD11b⁻CD2⁺ macrophages of the present invention is useful as a preventive and therapeutic agent for dermal lesions in promoting healing of folliculitis, urticaria pigmentosum, keloid, fibromatosis, anetoderma, striae atrophicae, senile atrophy, Kyrle disease, perforating folliculitis, sarcoidosis, foreign body granuloma, hyperhidrosis, sudamina, acne vulgaris of fingers, alopecia areata and the like, in preventing and improving of skin aging such as wrinkle, darkness, pigmentation and skin roughness and in improving skin tone, because it has a promoting effect on collagen synthesis in a dermal fibroblast, an activating effect on metabolism in a dermal fibroblast, a promoting effect on regeneration and proliferation of elastic fiber or glandular tissue or an angiogenesis induction effect. Still, in the repair and regeneration of dermal lesions, in addition to skin appendage such as papilla pili, collagen and elastic fibers, adipose tissue may also be included.

A pharmaceutical containing the compound which promotes induction of regeneration-promoting CD11b⁺CD2⁺ macrophages and regeneration-promoting CD11b⁻CD2⁺ macrophages used in the present invention is useful as a preventive and therapeutic agent for the overall skin diseases, because it is effective in either the above-described cutaneous epidermal or dermal lesions and in promoting healing of cut, trauma such as burn, ulcer, wound such as decubitus and post-operative surgical wound, which may lead both cutaneous epidermal and dermal lesions, and in lupus erythematosus, psoriasis, pigmentosa, bullous disease and the like.

Moreover, in the pharmaceutical of the present invention, the preventive and therapeutic agent for epidermal lesions and the preventive and therapeutic agent for dermal lesions may be used concomitantly.

When using the pharmaceutical of the present invention on skin diseases, it is especially preferable to use a pharmaceutical containing a compound which promotes induction of regeneration-promoting CD11b⁺CD2⁺ macrophages and regeneration-promoting CD11b⁻CD2⁺ macrophages, or to use a preventive and therapeutic agent for epidermal lesions and a preventive and therapeutic agent for dermal lesions together.

Upon combination use of the pharmaceuticals, for example, when using in kidney diseases, times of administering the preventive and therapeutic agent for renal glomerular lesions and the preventive and therapeutic agent for renal tubulointerstitial lesions have no limitation, and these may be administered to a subject simultaneously or may be administered separately in different time. Examples of such administering mode include: (1) an administration of a single formulation obtained by formulating the preventive and therapeutic agent for renal glomerular lesions and the preventive and therapeutic agent for renal tubulointerstitial lesions and optionally a pharmaceutical additive together; (2) a simultaneous administration via a same route of administration of two formulations obtained by separately formulating the preventive and therapeutic agent for renal glomerular lesions and optionally a pharmaceutical additive, and the preventive and therapeutic agent for renal tubulointerstitial lesions and a pharmaceutical additive as needed; (3) a separate administration in different times via a same route of administration of two formulations obtained by separately formulating the preventive and therapeutic agent for renal glomerular lesions and optionally a pharmaceutical additive, and the preventive and therapeutic agent for renal tubulointerstitial lesions and a pharmaceutical additive as needed; (4) a simultaneous administration via different routes of administration of two formulations obtained by separately formulating the preventive and therapeutic agent for renal glomerular lesions and optionally a pharmaceutical additive as needed, and the preventive and therapeutic agent for renal tubulointerstitial lesions and a pharmaceutical additive; and (5) a separate administration in different times via different routes of administration of two formulations obtained by separately formulating the preventive and therapeutic agent for renal glomerular lesions and optionally a pharmaceutical additive as needed, and the preventive and therapeutic agent for renal tubulointerstitial lesions and a pharmaceutical additive.

Daily dose of the pharmaceutical according to the present invention, which is varied depending upon diseases to be treated, administration route, dosage form, etc., is preferably about 0.1 to 100 mg/kg, more preferably about 1 to 50 mg/kg, although it cannot generally say. When the pharmaceutical according to the present invention is an external medicine, preferably it is prepared as containing an effective ingredient in 0.01 to 10% by weight.

As a compound formulated in the cosmetic of the present invention, any of the above-described compounds and pharmacologically acceptable salts thereof can be used. Specific examples of those include the compound which preferentially increases regeneration-promoting CD11b⁺CD2⁺ macrophages such as compounds (II-1) to (II-11) and (II-11a) and pharmacologically acceptable salts thereof such as those salts described above (e.g., sodium salt and potassium salt). As the cosmetic of the present invention has the same effects as of the preventive and therapeutic agent for epidermal lesions , for example, a promoting effect on a proliferation of melanocytes or epidermal cells, especially epidermal keratinocytes and a normalizing effect on epidermal cell function, it is preferably used for prevention and/or improvement of darkness, pigmentation and skin roughness.

Also, as the cosmetic in which the compound which preferentially increases regeneration-promoting CD11b⁻CD2⁺ macrophages such as compounds (I-1) to (I-4) and (I-4a) and pharmacologically acceptable salts thereof such as those salts described above (e.g., sodium salt and potassium salt) is formulated has the same effects as of the preventive and therapeutic agent for dermal lesions, for example, a promoting effect on collagen synthesis in a dermal fibroblast, an activating effect on metabolism in a dermal fibroblast, a promoting effect on regeneration and proliferation of elastic fiber or glandular tissue and an angiogenesis induction effect, it may be used for prevention and/or improvement of skin aging such as wrinkles and improvement of skin tone.

Further, as the cosmetic in which the compound which preferentially increases the regeneration-promoting CD11b⁺CD2⁺ macrophages and the regeneration-promoting CD11b⁻CD2⁺ macrophages such as compounds (III-1) and (III-2) and pharmacologically acceptable salts thereof such as those salts described above (e.g., sodium salt and potassium salt) is formulated has the same effects as prevention and therapy for epidermal lesions and dermal lesions, it may be used for prevention and/or improvement of skin aging such as wrinkles, darkness, pigmentation and skin roughness and improvement of skin tone.

As yet another embodiment of the cosmetic, there may be a cosmetic in which both induction-promoting compounds for the regeneration-promoting CD11b⁺CD2⁺ macrophages and for the regeneration-promoting CD11b⁻CD2⁺ macrophages are formulated. As still another embodiment, a cosmetic in which the induction-promoting compound for the regeneration-promoting CD11b⁺CD2⁺ macrophages is formulated and a cosmetic in which the induction-promoting compound for the regeneration-promoting CD11b⁻CD2⁺ macrophages is formulated may be used together. Concomitant use of the cosmetics and the cosmetic in which both compounds are formulated have the same effect as of the cosmetic in which the compound which promotes inductions of the regeneration-promoting CD11b⁺CD2⁺ macrophages and the regeneration-promoting CD11b⁻CD2⁺ macrophages. As the usage of the cosmetic, especially preferable are the combination use described above and the use of the cosmetic in which the both compound are formulated.

In the cosmetic of the present invention, in addition to the essential ingredient described above, an ingredient ordinary formulated in a cosmetic may be contained as needed. Examples of the ingredient formulated include an oil ingredient, a moisturizer, an emollient agent, an ultraviolet absorber, an antioxidant, a surfactant, an antiseptic, a complex lipid, an active oxygen scavenger, antiinflammatory agent, a vitamin and derivatives thereof, a circulation enhancer, an antiseborrheic agent, organic and inorganic pigments, botanical extract, animal extract, ingredient from a microorganism or algal extract anticipated to have some desirable effect on skin, a thickener, an amino acid, cholesterol, phytosterol, lipoprotein, a dye, a pH adjuster, a flavorant, a cool sensation-imparting agent, an antiperspirant, a bactericide, a skin-softener and water, which may be contained within the qualitative and quantitative range of not undermining the object and effect of the present invention.

Examples of the oil ingredient include avocado oil, palm oil, peanut oil, beef tallow, rice bran oil, rice germ oil, jojoba oil, ester oil, silicon oil, carnauba wax, lanolin, liquid paraffin, squalane, oxystearic acid, isostearic acid, isostearyl alcohol, cetyl alcohol, hexadecyl alcohol and the like.

Examples of the moisturizer include, glycerol, 1,3-butylene glycol, sorbitol, polyethylene glycol, collagen, hyaluronic acid and salt thereof, chondroitin sulfate and salt thereof, chitin, chitosan and the like.

Examples of the emollient agent include, long-chain acyl glutamic acid cholesteryl ester, cholesteryl hydroxystearate, 12-hydroxystearic acid, stearic acid, rosin acid, lanolin fatty acid cholesteryl ester and the like.

Examples of the ultraviolet absorber include, amyl paradimethylaminobenzoate, urocanic acid, ethyl diisopropylcinnamate and the like.

Examples of the antioxidant include sodium erythorbate, parahydroxyanisole and the like.

Examples of the surfactant include, sodium stearyl sulfate, diethanolamine cetyl sulfate, cetyltrimethyl ammonium chloride, polyethylene glycol isostearate, glycerol stearate and the like.

Examples of the antiseptic include ethylparaben, butylparaben and the like.

Examples of the complex lipid include glycerophospholipid, sphingophospholipid, glyceroglycolipid, sphingoglycolipid and the like.

Examples of the active oxygen scavenger include superoxide dismutase, catalase, β-carotene, oil-soluble licorice root extract, glabridin, licochalcone A, baicalin, baicalein, Ginkgo biloba leave extract, Sophorae Radix extract, Hamamelis virginiana extract, Engelhardtia extract and the like.

Examples of the antiinflammatory substance include allantoin, guaiazulene, chamazulene, bisabolol, glycyrrhizinic acid and salt thereof, glycyrrhetinic acid and salt thereof, stearyl ε-iminocaproate, indomethacin, zinc oxide, hinokitiol and the like.

Examples of the vitamin and derivatives thereof include retinol, retinal, retinoic acid, pantothenic acid, panthenol, riboflavin, pyridoxine, tocopherol, ascorbic acid, folic acid, nicotinic acid and the like.

Examples of the circulation enhancer include γ-oryzanol, dextran sodium sulphate and the like.

Examples of the antiseborrheic agent include sulfur, thianthol and the like.

Examples of the organic and inorganic pigments include: inorganic powder of such as silicic acid, silicic anhydride, magnesium silicate, talc, sericite, mica, kaolin, iron oxide red clay, bentonite, titanium-coated mica, bismuth oxychloride, zirconium oxide, magnesium oxide, zinc oxide, titanium oxide, aluminium oxide, calcium sulfate, barium sulfate, magnesium sulfate, calcium carbonate, magnesium carbonate, iron oxide, ultramarine blue, chromium oxide, chromium hydroxide, calamine, carbon black and composite thereof; organic powder of such as polyamide, polyester, polypropylene, polystyrene, polyurethane, vinyl resin, urea resin, phenol resin, fluorocarbon resin, silicone resin, acryl resin, melamine resin, epoxy resin, polycarbonate resin, divinylbenzene-styrene copolymer, silk powder, cellulose, CI pigment yellow, CI pigment orange; and composite powder of these inorganic and organic powders.

Examples of the botanical extract, animal extract, ingredient from a microorganism or algal extract anticipated to have some desirable effect on skin include Arnica montana extract, Artemisiae capillari extract, Scutellariae Radix extract, Phellodendri cortex extract, Coptidis rhizoma extract, Matricaria chamomilla (chamomile) extract, Glycyrrhizae radix water-extract, Gardeniae fructus extract, Lithospermum erythrorhizon extract, Paeoniae lactiflora extract, Paeonia suffruticosa extract, Houttuynia cordata extract, Betula platyphylla (white birch) extract, Aesculus hippocastanum (horse chestnut) seed extract, Calendula officinalis extract, Moutan cortex extract, Sapindus mukurossi extract, Tilia japonica(linden) extract, Rosmarinus officinalis (rosemary) extract, Achillea millefolium (yarrow) extract, Artemisia princeps (mugwort) extract, Bergenia purpurascens extract, Coix lachryma-jobi extract, aloe extract, loquat extract, peach extract, Cnidium officinale extract, Salvia officinalis (sage) extract, Angelica sinensis extract, luffa extract, Tilia europaea extract, Panax ginseng extract, capsicum extract, Rubus suavissimus extract, tea extract, Hypericum perforatum extract, Aesculus hippocastanum (horse chestnut) extract, prune extract, collagen, hydrolyzed conchiolin, elastin, vitronectin, fibronectin, placenta extract, royal jelly, bifidobacteria cultures, lactic acid bacteria cultures, yeast extract, Ganoderma lucidum mycelia extract, Poria cocos extract, brown algae extract, red algae extract, green algae extract and the like.

Examples of the thickener include cellulose derivative such as methyl cellulose and hydroxypropyl cellulose, carboxyvinyl polymer and the like.

Examples of the antiseptic include paraben such as methylparaben, quaternary ammonium salt such as benzalkonium chloride and the like.

The cosmetic of the present invention may be in a form such as solution, emulsion and paste mixture. Specific examples of the cosmetic of the present invention include facial washing cream, facial washing foam, cleansing cream, cleansing milk, cleansing lotion, massage cream, cold cream, moisture cream, skinmilk, skinlotion, facepack, foundation, after-shavecream, sunscreen cream, tanning oil, body shampoo, hair shampoo, hair rinse, hair treatment, hair nourishment composition, hair growth tonic, hair stick, hair cream, hair liquid, setting lotion, hair spray, hair dye, hair bleach, coloring rinse, coloring spray, permanent wave liquid, pressed powder, loose powder, eye shadow, hand cream and the like.

The method of using the cosmetic of the present invention, which is varied depending upon age, individual and site of application, is preferably the application to the skin from once to several times per day, of a cosmetic containing around 0.1 to 10% by weight, preferably around 0.1 to 5% by weight of the above-described compound, but not limited thereto.

The present invention provides a method for screening a compound which can be a pharmaceutical for preventing production and function of cytotoxic macrophages lesion-selectively causing a tissue disorder, inhibiting cell processes of causing apoptosis, effecting the repair and regeneration of lesions by inducing regeneration-promoting immunocompetent cells, or regulatory T lymphocytes and regeneration-promoting macrophages selectively to the immune response in lesions, recovering and saving organ tissue functions and preventing and/or treating a progressive disorder, or which can prevent, mitigate or treat lesions of cutaneous epidermis or dermis.

The method for screening can be conducted by determining whether a selective induction of regulatory T lymphocytes and regeneration-promoting macrophages corresponding to lesions are promoted or not.

More specifically, by determining whether a test compound promotes induction of regulatory CD2⁻CD4⁺ T lymphocytes and regeneration-promoting CD11b⁺CD2⁺ macrophages or not, a compound which promotes repair and regeneration of renal glomerulus and pancreatic islets of Langerhans can be screened. The compound which promotes induction can be a preventive and therapeutic agent for glomerular lesions and lesions of pancreatic islets of Langerhans. Further, by determining whether a test compound promotes induction of regulatory CD2⁻CD4⁺ T lymphocytes and regeneration-promoting CD11b⁺CD2⁺ macrophages or not, a compound which promotes repair, regeneration and/or proliferation of cutaneous epidermal cells or melanocytes can be screened. The compound which promotes induction can be a preventive and therapeutic agent for epidermal lesions. By determining only whether a test compound promotes induction of regeneration-promoting CD11b⁺CD2⁺ macrophages, the compound can also be screened.

By determining whether a test compound promotes induction of regulatory CD2⁻CD4⁺ T lymphocytes and regeneration-promoting CD11b⁻CD2⁺ macrophages or not, a compound which promotes repair and regeneration of renal tubulointerstitial lesions and lesions of pancreatic exocrine or interstitial tissues can be screened. The compound which promotes induction can be a preventive and therapeutic agent for renal tubulointerstitial lesions and lesions of pancreatic exocrine or interstitial tissues. Further, by determining whether a test compound promotes induction of regulatory CD2⁻CD4⁺ T lymphocytes and regeneration-promoting CD11b⁻CD2⁺ macrophages or not, a compound which promotes regeneration or proliferation of collagen, elastic fiber and/or glandular tissue, or angiogenesis can be screened. The compound which promotes induction can be a preventive and therapeutic agent for dermal lesions. By determining only whether a test compound promotes induction of regeneration-promoting CD11b⁻CD2⁺ macrophages, the compound can also be screened.

Any compound can be used in the method for screening of the present invention as a test compound, including peptide, protein, nonpeptidic compound, synthetic compound, fermentation product, cell extract, plant extract, animal tissue extract and plasma, which may be a known compound or a new compound.

It is also possible to combine known methods in such a manner that plural test compounds are screened at the same time and, only when there is a positive result, each compound is re-screened.

Below, for conducting the method for screening of the present invention, a method for inducing regulatory T-lymphocytes and regeneration-promotingmacrophages selectively against lesions is described. The following method is a preferable embodiment and does not limit the method for the induction.

Regeneration-promoting CD11b⁺CD2⁺ macrophages and regulatory CD2⁻CD4⁺ T lymphocytes which selectively repair and regenerate renal glomerular lesions, lesions of pancreatic islets of Langerhans or epidermal lesions are induced by contacting human peripheral blood mononuclear cells with lipopolysaccharide. The induction of regulatory CD2⁻CD4⁺ T lymphocytes can also be observed indirectly according to the following method. By cultivating human peripheral blood mononuclear cells in RPMI 1640 medium in the presence of lipopolysaccharide and human AB blood type serum, cytotoxic macrophages are induced. The induced cytotoxic macrophages are brought into contact with monolayered autologous erythrocytes. Upon this time, by adding peripheral blood T lymphocytes without resetting property with autologous erythrocytes, induction of regulatory CD2⁻CD4⁺ T lymphocytes can be observed via a number of produced cytotoxic macrophages. In this time, if a number of produced cytotoxic macrophages is decreased, regulatory T lymphocytes are induced, and the more decreased the number is, the more promoted the induction of regulatory T-lymphocytes is.

Regeneration-promoting CD11b⁻CD2⁺ macrophages and/or regulatory CD2⁻CD4⁺ T lymphocytes which repair and regenerate renal tubulointerstitial lesions, lesions of pancreatic exocrine or interstitial tissues or dermal lesions are induced by contacting human peripheral blood mononuclear cells with mitomycin-treated human peripheral blood mononuclear cells. Induction of the regulatory CD2⁻CD4⁺ T lymphocytes can also be observed indirectly according to the following method. By cultivating human peripheral blood mononuclear cells and mitomycin-treated human peripheral blood mononuclear cells in RPMI 1640 medium in the presence human AB blood type serum, cytotoxic macrophages are induced. The induced cytotoxic macrophages are brought into contact withmonolayered autologous erythrocytes. Upon this time, by adding peripheral blood T lymphocytes without resetting property with autologous erythrocytes, induction of regulatory CD2⁻CD4⁺ T lymphocytes can be observed via a number of produced cytotoxic macrophages. In this time, if a number of produced cytotoxic macrophages is decreased, regulatory T lymphocytes are induced, and the more decreased the number is, the more promoted the induction of regulatory T lymphocytes is.

Whether induction of regulatory T lymphocytes and regeneration-promoting macrophages is promoted by the presence of a test compound, which can be conducted as described above, can be decided by conducting the above-described induction in the presence or absence of a test compound, measuring numbers of produced regulatory T lymphocytes and regeneration-promoting macrophages according to a known method, and comparing the numbers of produced in the presence or absence of the test compound to determine whether the number of produced in the presence of test compound is increased or not. Examples of the known method include flow cytometry.

When observing induction of regulatory T lymphocytes via a number of produced cytotoxic macrophages, if a number of produce cytotoxic macrophages in the presence of a test compound is less than that in the absence of the test compound, the test compound can be considered as a desired compound effects on repair and regeneration of lesions.

A preferred embodiment of the method for screening of the present invention is described below.

Examples of the method for screening a compound which can prevent, mitigate or treat renal glomerular lesions, lesions of pancreatic islets of Langerhans or epidermal lesions include a method for screening a compound in the presence of which numbers of CD11b⁺CD2⁺macrophages and CD2⁻CD4⁺ T lymphocytes are increased by inducing regeneration-promoting CD11b⁺CD2⁺ macrophages and regulatory CD2⁻CD4⁺ T lymphocytes by cultivating human peripheral blood mononuclear leukocytes in RPMI 1640 medium in the presence of the test compound, lipopolysaccharide and human AB type serum, measuring numbers of CD11b⁺CD2⁺ macrophages and CD2⁻CD4⁺ T lymphocytes by flow cytometry, and comparing with the numbers in the absence of the test compound (for convenience, referred to as screening method A).

As the lipopolysaccharide, a lipopolysaccharide which has been known per se may be used. Examples of the lipopolysaccharide include lipopolysaccharides derived from gram-negative bacteria such as Salmonella and Escherichia coli. It may be the so-called rough type or smooth type.

For the preparation of the lipopolysaccharide, a method which has been known per se may be used, including, for example, a method of extracting from microorganism and removing toxicity as needed. Examples of the method of extracting from microorganism include a method of extracting with hot phenol (Westphal & Jann., Methods Carbohydr. Chem., 5, 83-89(1965)) and a method of treating the microorganism with proteinase K in the presence of sodium laurylsulfate (SDS). Alternatively, chemically synthesized lipopolysaccharide may be used or a commercially available one may be appropriately used. In the present invention, when a lipopolysaccharide is made into a solution using an appropriate solvent, preferably, RPMI 1640 liquid, it is preferred to use a solution of a high concentration of about 60 to 100 µg/ml, more preferably about 70 to 90 µm/ml or still more preferably about 80 µg/ml.

The human peripheral blood mononuclear cells can be obtained from human peripheral blood by a method known per se. Examples of a method of separating mononuclear cells from human peripheral blood include a centrifugation using Ficoll-Paque (registered trademark; manufactured by Pharmacia Fine Chemicals) which is a solution of 5.7% w/v of Ficoll 400 and 9.0% w/v of sodium diatrizoate adjusted to 1.077g/mL in specific gravity. More specifically, the method comprises the steps of: (a) placing a pre-determined amount of Ficoll-Paque at the bottom of a test tube; (b) transferring a blood sample as it is or diluted carefully onto the Ficoll-Paque with a pipette; (c) centrifuging the blood preparation on Ficoll-Paque prepared in (b) at about 400 to 500 × g for about 30 to 40 minutes so that a blood component having larger specific gravity than the specific gravity of Ficoll-Paque comes into or passes through Ficoll-Paque; and (d) collecting a mononuclear cell layer separated on the upper area of Ficoll-Paque with a pipette.

The RPMI 1640 medium has been described by Goding, J. W., in (1980) J. Immunol. Methods 39, 285, JAMA 199 (1957) 519. Alternatively, a commercially available product (manufactured by Sigma) may be used. A test compound, lipopolysaccharide, human AB type serum and human peripheral blood mononuclear cells may be added to the medium as a premixed combination of any two of or all of them, or may be added to the medium alone respectively. There is no limitation for the order of adding to the medium. More preferably, however, to RPMI 1640 medium, to which a test compound and human AB type serum have previously been added, human untreated peripheral mononuclear cells are added and followed by lipopolysaccharide.

The screening method A is, more specifically, conducted as follows. Human normal peripheral blood mononuclear cells (hereinafter, also abbreviated as PBMC) are separated by a specific gravity centrifugal method. About 40 ml of peripheral blood is collected with a heparin-added sterile syringe, and immediately transferred into 50 ml plastic tube containing EDTA-added (1.2 ml of EDTA per 100 ml) sterile cold saline. The blood is layered on layers of amount of Histopaque (d = 1. 111, manufactured by Sigma) and Ficoll-Paque (d = 1.078, manufactured by Pharmacia Biotech), and centrifuged to give a PBMC fraction. Lipopolysaccharide (hereinafter, also abbreviated as LPS) used is originated from Sigma L-4391 E-coli. To 2×10⁶ PBMC floated on RPMI1640 medium containing a test compound, human normal serum of type AB, 2 mM of L-glutamine, 5 µg/mL of gentamicin, LPS is added so as to make a concentration 80 µg/mL, and the PBMC is cultivated for 6 days at 37°C under 5% CO₂-95% air. At this time, the concentration of the test compound has no limitation, but is preferably about 1 µM to 0.001 µM. Also preferably, the test compound is added at various concentrations.

After cultivating, all of adherent cells are collected and floated on a Hanks solution containing 1% of bovine albumin. For flow cytometry, the following labeled anti-human CD2, CD4 and CD11b monoclonal antibodies are used, that is, an anti-human CD2 monoclonal antibody (SFCI3Pt2H9 (T11-1), manufactured by Coulter) labeled with fluorescein isocyanate (manufactured by FITC), an anti-human CD4 monoclonal antibody (allophycocyanin-labeled antibody, manufactured by Beckton Dickinson Immunocytometry Systems), and an anti-human CD11b monoclonal antibody (clone 44, manufactured by GenzymeTechne) labeled with phycoerythrin. By using FACScan, fractions of CD11b⁺CD2⁺ monocyte macrophages and CD2⁻CD4⁺ T lymphocytes are measured. When a fraction is increased by not less than 20% by adding a test compound, the compound is considered as positive.

Examples of the method for screening a compound which can prevent, mitigate or treat renal tubulointerstitial lesions, lesions of pancreatic exocrine or interstitial tissues or dermal lesions include a method for screening a compound in the presence of which numbers of CD11b⁻CD2⁺ macrophages and CD2⁻CD4⁺ T lymphocytes are increased, by inducing regeneration-promoting CD11b⁻CD2⁺ macrophages and/or regulatory CD2⁻CD4⁺ T lymphocytes by cultivating human peripheral blood mononuclear cells and mitomycin-treated human peripheral blood mononuclear cells in RPMI 1640 medium in the presence of the test compound and human AB type serum, measuring numbers of CD11b⁻CD2⁺ macrophages and CD2⁻CD4⁺ T lymphocytes by flow cytometry, and comparing with the numbers in the absence of the test compound (for convenience, referred to as screening method B).

The mitomycin-treated human peripheral mononuclear cells, for example, can be obtained by adding mitomycin to human peripheral mononuclear cells obtained by the above-described known method so as to make a final concentration of mitomycin about 40 µg/mL, and heating for about 20 minutes at about 37°C.

More specifically, a screening method B can be conducted similarly as a screening method A, except that human peripheral blood mononuclear cells are cultivated with same number of mitomycin-treated human peripheral blood mononuclear cells instead of LPS. In other words, on RPMI1640 medium containing a test compound, human normal serum of type AB, 2 mM of L-glutamine and 5 µg/mL of gentamicin, 2×10⁶ PBMC and 2×10⁶ mitomycin-treated PBMC are floated, and cultivated for 6 days at 37°C under 5% CO₂-95% air. Cultivation conditions and labeled antibodies used in flow cytometry are same as in the screening method A, and a determination of a positive compound is also similarly conducted.

Another embodiment of the method for screening a compound which can prevent, mitigate or treat renal glomerular lesions, lesions of pancreatic islets of Langerhans or epidermal lesions include a method for screening a compound in the presence of which a number of produced cytotoxic macrophages is decreased, comprising the steps of (i) inducing cytotoxic macrophages by cultivating human peripheral blood mononuclear cells in RPMI 1640 medium in the presence of lipopolysaccharide and human AB type serum; (ii) at the same time, inducing unfractionated T lymphocytes by cultivating human peripheral blood unfractionated T lymphocytes with or without lipopolysaccharide in the presence of the test compound; (iii) bringing the cytotoxic macrophages obtained in (i) into contact with monolayered autologous erythrocytes in the presence of the unfractionated T lymphocytes obtained in (ii), and measuring a number of produced cytotoxic macrophages; and (iv) comparing with the number of generated cytotoxic macrophages in the absence of the test compound (for convenience, referred to as screening method C).

Specifically, the method can be conducted according to the following steps.
(a) Induction of cytotoxic macrophages is described below. To 2×10⁶ PBMC floated on RPMI1640 medium containing human normal serum of type AB, 2 mM of L-glutamine and 5 µg/mL of gentamicin, LPS is added so as to make a concentration 80 µg/mL, and the PBMC is cultivated for 6 days at 37°C under 5% CO₂-95% air to induce cytotoxic macrophages. The PBMC is obtained similarly as in the screening method A. The induced cytotoxic macrophages are recovered from the medium. In the recovering, a known method may be used, including a method of collecting adhered materials with a rubber-policeman (rubber spatula). Then, the recovered cytotoxic macrophages may be washed. In washing, a Hanks solution with about 5 µg/mL of gentamicin is preferably used.
(b) Induction of unfractionated T lymphocytes is described below. First, human peripheral blood unfractionated T lymphocytes is separated as described below. As described in the screening method A, to a PBMC fraction separated by a specific gravity centrifugal method was added sheep erythrocytes treated with neuraminidase and reacted for 20 minutes at 37°C. This solution is gently added to a tube in which Percolls of discontinuous specific gravities are layered, and centrifuged at 400xg for 20 minutes at room temperature. In the layered Percolls of discontinuous specific gravities, fractions of rosetted T lymphocytes and monocytes floating on a layer of not less than d = 1.091 are collected respectively. To the fraction of rosetted T lymphocytes is added ammonium chloride to hemolyze the sheep erythrocyte and a fraction of T lymphocytes is obtained. The fraction of monocytes obtained at the same time is washed.
   Next, by cultivating the separated human peripheral blood unfractionated T lymphocytes with or without lipopolysaccharide in the presence of a test compound, unfractionated T lymphocytes can be induced. Specifically, in RPMI 1640 medium containing human normal serum of type AB, 2 mM of L-glutamine, 5 µg/mL of gentamicin and lipopolysaccharide as needed, human peripheral blood unfractionated T lymphocytes are cultivated for 6 days at 37°C under 5% CO₂-95% air. The induced unfractionated T lymphocytes are recovered from the medium. In the recovering, a known method may be used, including a method of collecting adhered materials with a rubber-policeman.
(c) With the cytotoxic macrophages obtained in (a) and the unfractionated T lymphocytes obtained in (b), a tissue-regenerating function of a test compound is estimated by SPFC method.

SPFC method is described below. The method is preferably conducted by contacting the cytotoxic macrophages obtained in (a) with monolayered autologous erythrocytes in the presence of the unfractionated T lymphocytes obtained in (b), and counting a number of produced Spontaneous Plaque-Forming Cell (hereinafter, referred to as SPFC).

First, monolayered autologous erythrocytes are prepared. Monolayered erythrocytes may be prepared by a method which has been known per se, preferably by the following method. To autologous erythrocytes are added a Hanks solution without serum supplementation to a concentration of about 4%. It is preferred to use autologous erythrocytes which are prepared by the above-mentioned known method and preserved at about 4°C in a phosphate-buffered saline (hereinafter, referred to as PBS) with 0.1% by weight of AB serum. To a Terasaki plate is added poly-L-lysine, treated for about 20 minutes at about 37°C, washed with PBS, and immediately added the autologous erythrocytes and allowed to stand for about 30 minutes at about 37°C. Then erythrocytes not adhered are removed to give a Terasaki plate to which monolayered autologous erythrocytes are adhered.

Cultivated PBNC containing the cytotoxic macrophages obtained in (a) is adjusted to a concentration of cell numbers about 2×10⁶/mL by adding a Hanks solution containing about 5 µg/mL of gentamicin.

Then, to a well of the Terasaki plate to which the monolayered autologous erythrocytes are adhered, about 1×10⁴ of the cytotoxic macrophages obtained in (a) are added. At the same time, the unfractionated cultivated T lymphocytes obtained in (b) are also added. To determine whether regulatory T-lymphocytes inhibiting SPFC activity by the cytotoxic macrophages are produced in the cultivated T lymphocytes or not, a ratio of cell numbers in two steps of the case of adding the unfractionated cultivated T lymphocytes obtained in (b) in same numbers as of the cytotoxic macrophages obtained in (a), and the case of adding the unfractionated cultivated T lymphocytes obtained in (b) in one-fifth numbers of the cytotoxic macrophages obtained in (a) is set. Finally, to the well is added a Hanks solution in 1 to 10 µL, and allowed to stand for about two hours at about 37°C. After completion of the reaction, it is preferred to fix by formalin. Numbers of the produced SPFC can be easily measured by a phase-contrast microscope. To determine whether a number of produced SPFC is suppressed or not, a suppression rate % is calculated by conducting same procedures only with a solvent without adding a test compound, and comparing with the result of adding the test compound. A compound expressing a suppression of not less than 50% is considered as positive.

Another embodiment of the method for screening a compound which can prevent, mitigate or treat renal tubulointerstitial lesions, lesions of pancreatic exocrine or interstitial tissues or dermal lesions include a method for screening a compound in the presence of which a number of produced cytotoxic macrophages is decreased comprising the steps of (i) inducing cytotoxic macrophages by cultivating human peripheral blood mononuclear cells and mitomycin-treated human peripheral blood mononuclear cells in RPMI 1640 medium in the presence of human AB type serum; (ii) at the same time, inducing unfractionated T lymphocytes by cultivating human peripheral blood unfractionated T lymphocytes with or without mitomycin-treated human peripheral blood mononuclear cells in the presence of a test compound; (iii) bringing the cytotoxic macrophages obtained in (i) into contact with monolayered autologous erythrocytes in the presence of the unfractionated T lymphocytes obtained in (ii), and measuring a number of produced cytotoxic macrophages; and (iv) comparing with the number of generated cytotoxic macrophages in the absence of the test compound (for convenience, referred to as screening method D).

Specifically, the screening method may be conducted similarly as the screeningmethod C except that mitomycin-treated PBMC is used instead of LPS.

Yet another preferable embodiment of the method for screening a compound which can prevent, mitigate or treat renal glomerular lesions, lesions of pancreatic islets of Langerhans or epidermal lesions include a method for screening a compound in the presence of which a number of regulatory CD2⁻CD4⁺ T lymphocytes is increased comprising the steps of (i) inducing unfractionated T lymphocytes by cultivating human peripheral blood mononuclear cells in the presence of a test compound, human AB type serum and lipopolysaccharide; (ii) measuring a number of regulatory CD2⁻CD4⁺ T lymphocytes in the induced unfractionated T lymphocytes by flow cytometry; and (iii) comparing with the result in the absence of the test compound (for convenience, referred to as screening method E).

Specifically, this screening method may be conducted according to the screening methods A to D.

Yet another preferable embodiment of the method for screening a compound which can prevent, mitigate or treat renal tubulointerstitial lesions, lesions of pancreatic exocrine or interstitial tissues or dermal lesions include a method for screening a compound in the presence of which a number of regulatory CD2⁻CD4⁺ T lymphocytes is increased comprising the steps of (i) inducing unfractionated T lymphocytes by cultivating mitomycin-treated human peripheral blood mononuclear cells and human peripheral blood mononuclear cells in the presence of a test compound and human AB type serum; (ii) measuring a number of regulatory CD2⁻CD4⁺ T lymphocytes in the induced unfractionated T lymphocytes by flow cytometry; and (iii) comparing with the result in the absence of the test compound (for convenience, referred to as screening method F).

Specifically, this screening method may be conducted according to the screening methods A to D.

The present invention further provides a screening kit for carrying out the above-mentioned screening method according to the present invention. There is no particular limitation for the form of the screening kit, and the forms which have been known per se may be used.

As a preferred embodiment of a screening kit for screening a compound which can prevent and/or treat renal glomerular lesions, lesions of pancreatic islets of Langerhans or epidermal lesions, included is a kit comprising (a) human peripheral blood mononuclear cells and (b) lipopolysaccharide. The kit may comprise those other than (a) and (b) described above such as (c) human AB type serum and/or (d) RPMI 1640 medium.

As a preferred embodiment of a screening kit for screening a compound which can prevent and/or treat renal tubulointerstitial lesions, lesions of pancreatic exocrine or interstitial tissues or dermal lesions, included is a kit comprising (a) human peripheral blood mononuclear cells and (b) mitomycin-treated human peripheral blood mononuclear cells. The kit may comprise those other than (a) and (b) described above such as (c) human AB type serum and/or (d) RPMI 1640 medium.

### BEST MODE FOR CARRYING OUT THE INVENTION

Below, the present invention is further described in detail by referencing Examples and Formulation Examples , but the present invention should not be limited by these Examples and Formulation

### EXAMPLES.

In the following Examples 1 to 3, as a test compound, (R)-1-naphthalen-2-ylethyl (S)-2-(4-fluorophenoxy)-5-oxotetrahydrofuran-2-carboxylate (compound (I-1)), 1-chloro-3-oxo-1,3-dihydroisobenzofuran-1-carboxylic acid benzyl ester (compound (I-3)), (R)-1-naphthalen-2-ylethyl (R)-2-(4-fluorophenoxy)-5-oxotetrahydrofuran-2-carboxylate (compound (II-1)), 2-fluoro-5-oxotetrahydrofuran-2-carboxylic acid benzyl ester (compound (II-2)) and 2-(4-chlorophenyl)thio-5-oxo-2-tetrahydrofuran carboxylic acid benzyl ester (compound (III-1)) were used.

### <Example 1>

Compound inducing production of regulatory CD2⁻CD4⁺ T lymphocytes

### Method:

The example was conducted according to the screening method C, except that not only a monocyte fraction but same T lymphocyte fraction was also obtained from normal human peripheral blood. For induction of cytotoxic macrophages, the monocyte fraction was cultivated for 6 days without adding mitogen. Compound (III-1) was dissolved in ethanol as a solvent in three concentrations of 10 µg/mL to 0.1 µg/mL as a final concentration at the start of cultivating, and added to the T lymphocyte fraction. The fractions were cultivated similarly for 6 days. At the end of cultivating, monocytes and T lymphocytes including adherent cells were collected.

To determine whether or not regulatory T lymphocytes which inhibit SPFC activity by cytotoxic macrophages were produced in the cultured T lymphocytes treated with compound (III-1), according to SPFC method, about 1×10⁴ of the cultured monocytes were added to a well in a Terasaki plate to which said monolayered autologous erythrocytes were adhered, and simultaneously five-fold the number of the cultured T lymphocytes treated with compound (III-1) were added. The mixture was allowed to stand for about two hours at around 37°C.

As shown in Table 1, by treating the T lymphocytes with not less than 0.1 µg/mL in concentration, not less than 50% of inhibition of the activity of the cytotoxic macrophages was resulted.

Among human T lymphocytes, a cell surface marker of an autorosette-forming T lymphocytes (ARFC-T) lymphocyte being able to rosette with autologous erythrocytes is CD2⁺, and a cell surface marker of a non-autorosette-forming T lymphocytes (non-ARFC-T) is CD2⁻. As the non-ARFC-T has a cell proliferation activity, CD4⁺ may be suggested.

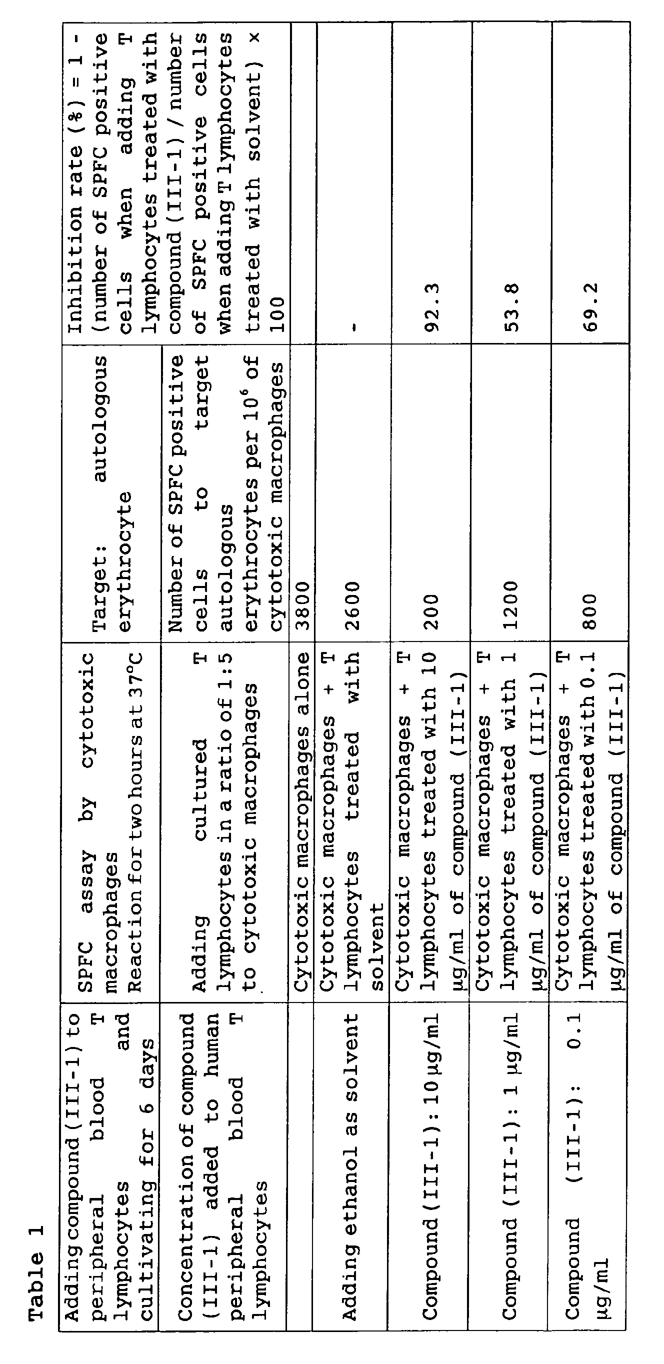

As described above, compound (III-1) is a compound without selectivity for induction of cytotoxic macrophages, but induces regulatory CD2⁻CD4⁺ T lymphocytes.

### <Example 2-1>

Evaluation of compound showing induction effect for regeneration-promoting macrophages among lesion-selective immunomodulating regeneration-promoting cells in animal experiments - study with obstruction release model after complete obstruction of unilateral ureter for 14 days.

### Method I:

Experimental models were prepared by a method devised and established by Ishibashi (Michio Ishibashi, et al. : The Japanese Journal of Nephrology, 42:248, 2000) using male SD rats of 8-9 weeks age and about 280 g. That is, the rat was laparotomized under anesthesia with ether and left ureter was ligated with 7-0 Nylon at the height of the margin of lower pole of kidney to close the abdomen. On the 14th day after obstruction, the obstruction was released and urinary passage was reconstructed using a cuff. Thus, after 14 days, the part of ligated obstructed ureter was resected, a polyethylene tube of 25 gages (manufactured by Nippon Sherwood) was used as a cuff and inserted into and retained at the lumen from the cut end of the lower normal ureter, then the cuff was also retained in the expanded upper ureter and each of them was ligated and fixed by 7-0 Nylon to reconstruct the urinary passage. At the same time, the right kidney at the opposite side was excised. After the release of the obstruction, body weight was measured, blood was drawn on the 2nd day, 5th day and 7th day from the release to measure serum creatinine, then the rat was killed under anesthesia and the left obstruction-released kidney was excised.

With regard to the excised kidney, there were carried out measurements of weight of the kidney, creatinine concentration mg/dl in plasma as an indicator of kidney function and pathological test for the kidney, and also leukocyte cells which infiltrate the obstruction-released kidney were immunopathologically studied with cell surface markers for ED1 macrophages, CD11b (ED8) positive macrophages and CD5 positive lymphocytes. A number of positive cells was expressed as a number of cells per glomerulus from measured numbers of positive cells in five glomeruli, and for a number in tubulointerstitial tissue, it was expressed by the total number of cells existing in five fields of 200×magnification. Formacrophages, a number of CD11b positive cells existing in glomerulus was considered as a number of CD11b⁺CD2⁺ macrophages and a case of existing not less than one positive cell per glomerulus was considered as positive (+). In the tubulointerstitial tissue, a number of not more than 60 CD11b positive cells was considered as positive (+), or a case of CD11b⁻CD2⁺ macrophages dominantly increasing. Scoring of evaluation of tubulointerstitial lesions was as follows: + gets 1-point as a light lesion; ++ gets 2-point as a moderate lesion; and +++ gets 3-point as a severe lesion. For glomerular lesions, when a proportion of lesioned glomeruli was decreased to not more than 40%, and for tubulointerstitial lesions, when a score of the lesions was not more than 4, it was considered as improved.

When the compound of the present invention was not administered to the model as described below, in pathomorphological observations over time during two weeks of the obstructed period and after release of the obstruction, there were fibrosis and atrophy, mainly composed of tubulointerstitial lesions of renal structure, and enlargement of the regenerated kidney accompanying repair process by the release of the obstruction. For a cell infiltration observed during the obstructed period, CD5 positive T cells were increased less significantly than macrophages, and ED1 positive macrophages became dominant on 10th day.

For glomerular lesions, by counting glomeruli causing thickening of Bowman's capsule wall and glomerular hyalinosis, a proportion of glomeruli having lesions was calculated. For tubulointerstitial lesions, by qualifying hyperplasia of tubular basement membrane and interstitial fibrosis, levels of the lesions were represented.

### Method II:

In a group of compounds (hereinafter, referred to as group II compound) inhibiting production of cytotoxic macrophages, which affect selectively renal glomerular lesions characterized by being induced by contacting human peripheral blood mononuclear cells with lipopolysaccharide in a test tube, there are known compound (II-1) and compound (II-2).

In a group of compounds (hereinafter, referred to as group I compound) inhibiting production of cytotoxic macrophages, which affect selectively renal tubulointerstitial lesions characterized by being induced by contacting human peripheral blood mononuclear cells with mitomycin-treated human peripheral blood mononuclear cells in a test tube, there is known compound (I-1).

An in vivo biological test was carried out for the γ-lactone derivative according to the present invention using this model. Bulk powder of a test compound was dissolved in sterile physiological saline together with gum arabic, and concentrations of the test compound and gum arabic were adjusted to 30 mg/mL and 5% respectively. The preparation was administered orally or subcutaneously injected at the dose of 30 mg/kg or 3 mg/kg every day. The administration was conducted for the total 21 days of the obstructed period for 14 days and after release of the obstruction for 7 days.

As a test compound, there was used compound (I-1) as the compound of group I suppressing the SPFC production by allo-MLC stimulation, i.e., the compound which selectively suppress the induction of effector macrophage in the presence of allo-MLC, and there were used (II-1) and (II-2) as the compounds of group II suppressing the SPFC production by LPS stimulation, i.e., the compounds which selectively suppress the induction of effector macrophage in the presence of LPS.

For test groups, a group administered single compound group, a group administered both compound group and a control group administered solution of gum arabic were set up and compared for renal functions and morphological evaluations, as well as numbers of ED1, CD5, CD11b positive cells by means of immunopathology.

The results are listed in Table 2-1.

In the control group It is seen that there were regeneration-promoting CD11b⁺CD2⁺ macrophages in glomeruli, and regeneration-promoting CD11b⁻CD2⁺ macrophages in tubulointerstitial lesions. However, it should be noted as an important result that there was no animal expressing a response of coincident increase of both regeneration-promoting macrophages confirmed in each lesions in the control group. For a level of lesions in the control group, there were lesions in 54% to 83% of glomeruli, and there were 7.5 to 11 of quality level of hyperplasia of tubular basement membrane and interstitial fibrosis in tubulointerstitial lesions.

First, compound (I-1) of the group I compound is a known compound and is known to inhibit induction of cytotoxic macrophages which selectively induce tubulointerstitial lesions. As seeing whether lesion-repair and regeneration CD11b⁻CD2⁺ macrophages to lesions were induced selectively in I-treated groups by this compound (I-1) of the group I compound or not, there were increased regeneration-promoting macrophages to tubulointerstitial lesions in three of four cases, while there were increased regeneration-promoting macrophages to glomerular lesions in two of four cases, which was similar as in the control group. For pathological improvement of lesions, only tubulointerstitial lesions were improved in three of four cases and no glomerular lesion was improved. There was no difference in serum creatinine.

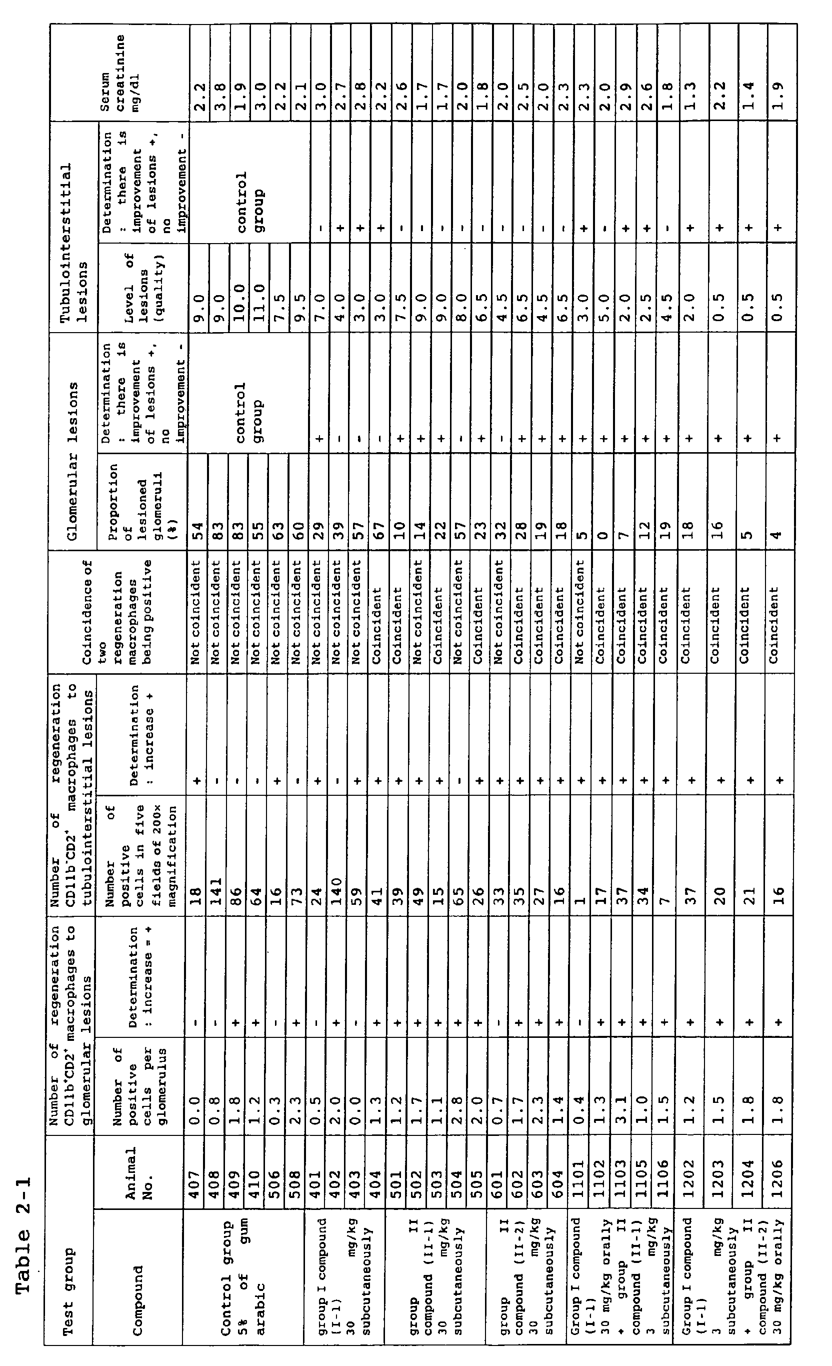

Compound (II-1) and compound (II-2) of the group I I compound are known compounds and are known to inhibit induction of cytotoxic macrophages which selectively induce tubulointerstitial lesions. In groups treated with compound (II-1) of the group II compound, there were increased regeneration-promoting CD11b⁺CD2⁺ macrophages to glomerular lesions in all five cases, and additionally, there were increased repair and regeneration CD11b⁻CD2⁺ macrophages to tubulointerstitial lesions in four of five cases. By group II compound, differing from group I compound, macrophages which repair and regenerate other lesions were induced. For pathological improvement of lesions, glomerular lesions were improved in four of five cases and no tubulointerstitial lesion was improved, that is, there was selectivity.

Effects on the group administered with compound (II-2) of the group II compound were similar as of the compound (II-1) of the group II compound. Regeneration-promoting macrophages were increased on both lesions, and in improvement of lesions, there was similar selectivity on improvement of glomerular lesions as of the compound (II-1) of the group II compound.

For the above results, in a compound represented by compound (I-1) of the group I compound, there was selectivity as induction of regeneration-promoting macrophages was consistent with improvement of lesions, but in two compounds of group II compound, although there was correlation on selectivity with improvement of glomerular lesions, there was no correlation between inductions of regeneration-promoting macrophages to glomerular lesions and to tubulointerstitial lesions. Accordingly, difference in phenotypes for CD87, i.e., uPAR (receptor of urokinase plasminogen activator) is suggested, and there can be CD11b⁻CD2⁺CD87- for regeneration-promoting macrophages to glomerular lesions and CD11b⁻CD2⁺CD87+ for regeneration-promoting macrophages to tubulointerstitial lesions.

Further, based on the above results, we examined whether selectivity for glomerular lesions and tubulointerstitial lesions is more obvious or not when group I compound and group II compound are used together.

In two test groups, one co-administered group was administered with compound (I-1) of the group I compound orally and compound (II-1) of group II compound subcutaneously in one-tenth amount and the other co-administered group was administered with compound (II-1) of group II compound orally and compound (I-1) of the group I compound subcutaneously in one-tenth amount, consistent cases of regeneration-promoting macrophages in both lesions, glomerular lesions and tubulointerstitial lesions were increased from 8 to 9 cases, and further, for improvement of both glomerular lesions and tubulointerstitial lesions, there were improvement of glomerular lesions in all cases and there were improvement of tubulointerstitial lesions in seven of nine cases. Especially, in the co-administered group administered with compound (II-1) of group II compound orally and compound (I-1) of the group I compound subcutaneously in one-tenth amount, an excellent effect was observed.

### <Example 2-2>

Evaluation of compound showing an induction effect for regeneration-promoting macrophages among lesion-selective immunomodulating regeneration-promoting cells in animal experiments- study with obstruction release model after complete obstruction of unilateral ureter for 14 days.

### Method I:

Experiment was conducted similarly as in method I of Example 2-1. For cell surface markers of leukocyte cells which infiltrate the obstruction-released kidney, ED1, CD11b (ED8), CD5 and CD2 were used. Counting of numbers of positive cells and CD11b positive cells and assessments were conducted similarly as in method I of Example 2-1. For CD5⁺, a number of not less than 20 per glomerulus was considered as positive. For glomerular lesions, which was characterized by Bowman's capsule wall, glomerular hyalinosis, dilation of urinary space pole and hypertrophy of parietal cells, 50 glomeruli were evaluated to obtain a proportion of pathologic glomeruli. For tubulointerstitial lesions, levels of the lesions were represented by qualifying hyperplasia of tubular basement membrane, tubular enlargement and atrophy and interstitial fibrosis. Scoring was as follows: + gets 1-point as a light lesion; ++ gets 2-point as a moderate lesion; and +++ gets 3-point as a severe lesion. Levels of cell infiltration were evaluated qualitatively according to the above three-point scale. For glomerular lesions, when a proportion of lesioned glomeruli was decreased to not more than 20%, and for tubulointerstitial lesions, when a score of the lesions was not more than 6.5, it was considered as improved.

### Method II:

Experiment was conducted similarly as in method II of Example 2-1, except that test groups were a group administered with compound I-3 (group I compound) alone, a group administered with compound II-1 (group II compound) alone and a group co-administered with compound I-3 and compound II-1.

Numbers of cells positive for CD11b (ED8), CD2 and CD5 were compared by means of immunopathology, and CD11b⁺CD2⁺ macrophages were considered as regeneration-promoting macrophages in glomeruli and CD11b-CD2⁺CD5⁺ macrophages were considered as regeneration-promoting macrophages to tubulointerstitial lesions, and herewith, it was estimated whether each cell group Is quantitatively dominant or not.

The results are listed in Table 2-2.

In the control group, a number of animals in which regeneration-promoting CD11b⁺CD2⁺ macrophages were predominant in glomeruli was two of six, a number of animals in which regeneration-promoting CD11b-CD2⁺CD5⁺ macrophages were predominant in tubulointerstitial lesions was one of six, but there was no animal expressing a response of coincident increase of both regeneration-promoting macrophages confirmed in each lesions. For a level of lesions in the control group, there were lesions in 28% to 52% of glomeruli, and there were 5.5 to 9.0 of level of tubulointerstitial lesions. In the group administered with compound I-3 of the group I compound, lesion-selective repair and regeneration-promoting CD11b⁻CD2⁺CD5⁺ macrophages to tubulointerstitial lesions were predominant in tubulointerstitial lesions in three of four cases, and regeneration-promoting CD11b⁺CD2⁺ macrophages were predominant in glomeruli in three of four cases. For pathological improvement of lesions, tubulointerstitial lesions were improved in two of four cases and glomerular lesion were improved in all four cases. There was no difference in serum creatinine. Meanwhile, in the group administered with compound II-1 of the group II compound, regeneration-promoting CD11b⁺CD2⁺ macrophages were predominant in glomerular lesions in all five cases, but regeneration-promoting CD11b⁻CD2⁺CD5⁺ macrophages were negative in tubulointerstitial lesions in all cases and there were no change. For pathological improvement of lesions, tubulointerstitial lesions were improved in all five cases having the lesions but glomerular lesion was improved in only one case. This suggests that macrophages have selectivity on repair and regeneration of lesions.

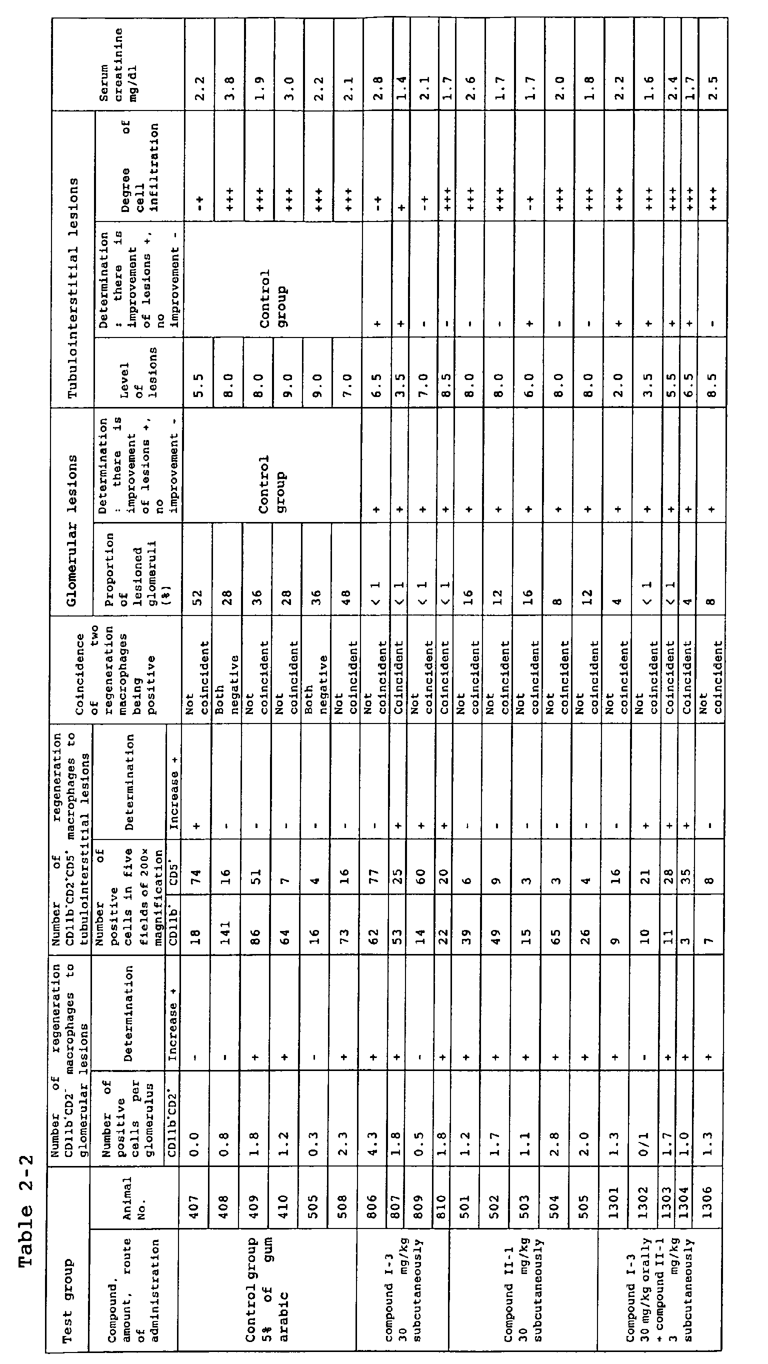

For selectivity to repair and regeneration of lesions, it is suggested that, when untreated, tubulointerstitial lesions have more various cytokine gradients than glomerular lesions and glomerular lesions are promoted, but compound I-3 of the group I compound may improve glomerular lesions by selectively inducing regeneration-promoting CD11b-CD2⁺CD5⁺ macrophages in tubulointerstitial lesions to resolve cytokine gradients between tubulointerstitial lesions and glomerular lesions, or by inducing-secreting a certain cytokine which induces regeneration-promoting CD11b⁺CD2⁺ macrophages. Meanwhile, in the case of compound II-1 which repairs and regenerates glomerular lesions, it was found that there was no cytokine gradient between glomerular lesions and tubulointerstitial lesions, and the compound was not involved in improvement of tubulointerstitial lesions.

Next, we examinedwhether selectivity for glomerular lesions and tubulointerstitial lesions is more obvious or not when compound I-3 of the group I compound and compound II-1 of the group II compound are used together.

In co-administered group receiving compound I-3 of the group I compound orally and compound II-1 of the group II compound subcutaneously in one-tenth amount, consistent cases of regeneration-promoting CD11b-CD2⁺CD5⁺ macrophages in both lesions, glomerular lesions and tubulointerstitial lesions were in two of five cases, and further, for improvement of both glomerular lesions and tubulointerstitial lesions, there were improvement of glomerular lesions in all cases and improvement of tubulointerstitial lesions in four of five cases. It is noted that there was no difference in degrees of cell infiltration between the control group and the co-administered group It is thought as that by co-administering compound I-3 of the group I compound and compound II-1 of the group II compound, regeneration-promoting macrophages became dominant in quantity.

From these results, it was found that a compound which inhibits production of cytotoxic macrophages selectively induced in glomerular lesions and tubulointerstitial lesions also have an effect of inducing phenotypes of regeneration-promoting macrophages selective for corresponding lesions.

### <Example 3>

Evaluation of compound showing an induction effect for regeneration-promoting macrophages among lesion-selective immunomodulating regeneration-promoting cells in animal experiments - study with complete skin-excised model

### Method:

Using male SDT rats of 28-30 weeks age, which were a spontaneously type II diabetes model, promoting effect on repair and regeneration in wound healing after complete skin excision were examined according to a partially modified method of Breitbout AS et al. (Ann Plast Surg 1999, 43: 632). That is, an animal was shaved at its back under anesthesia with ether, and skin in 2 × 2 cm size was excised together with panniculus carnosus in same size to make two complete skin-excised wounds per animal.

Healings of the wounds were observed on the 1st day, 4th day, 7th day, 12h day, 15th day, 21st day and 28th day from the operation. An area of the wounds was obtained as the product of the average vertical length and the average horizontal length which were measured along with a direction of the longest part in the wound. In the complete skin-excised model, activity of a test compound for repair and regeneration of dermis and epidermis can be revealed by examining degrees of reduction of the wound area and generation of scar.

Using this model, we examined whether group I compound (I-1) and group II compound (II-2) which exhibit biological effects in <Example 2> have biological effects on healing of a complete skin-excised wound or not. A test compound was dissolved in sterile physiological saline together with gum arabic. Concentrations of the test compound and gum arabic were adjusted to 30 mg/mL and 5% respectively. The preparation was subcutaneously injected at the dose of 10 mg/kg every day. The administration was started on 6 days before the operation and continued to 28 days after the operation.

### Result:

The results are shown in Table 3. Group I compound (I-1) showed more promoting effect on wound healing at 7th day to 12th day after the operation than that in the control group and in Group II compound (II-1). Also in 28th day of healing period, it showed promoting effect on wound healing. Group II compound (II-1) showed a significant promoting effect on wound healing at 12th day after the operation compared with the control group In Table, 1) represents a number of wounds (two excised wounds per animal × number of test animals), 2) represents the post-operative day after complete skin excision. * means a significant difference (p > 0.05) from a group administered with 5% gum arabic, and ** means a significant difference (p > 0.01) from the group administered with 5% gum arabic.

**Table 3**

| Test group | n¹⁾ | Excised wound area (cm²) | | | | | | |
|---|---|---|---|---|---|---|---|---|
| | | 1^{st} day²⁾ | 4^{th} day²⁾ | 7^{th} day²⁾ | 12^{th} day²⁾ | 15^{th} day²⁾ | 21^{st} day²⁾ | 28^{th} day²⁾ |
| 5% of gum arabic | 10 | 3.29 | 2.37 | 1.77 | 0.79 | 0.58 | 0.53 | 0.32 |
| Compound (I-1) | 8 | 3.38 | 2.10 | 1.45 * | 0.56* | 0.51 | 0.40 | 0.18* |
| Compound (II-1) | 8 | 3.33 | 2.31 | 1.62 | 0.52^{*} * | 0.55 | 0.44 | 0.29 |

From Table 4, at 28th day reaching to complete healing, in the control group, 50% of repaired wounds occurred crust formation on its surface, but in group I compound (I-1) and group II compound (II-1), no crust formation occurred and wound surface was finely healed.

**Table 4**

| | Crust formation / number of wound | |
|---|---|---|
| | 21st day after the operation | 28th day after the operation |
| 5% of gum arabic | 5/10 | 5/10 |
| Compound (I-1) | 0/8 | 0/8 |
| Compound (II-1) | 0/8 | 1/8 |

From these results, it was found that group I compound (I-1) has a promoting effect on healing of dermis and epidermis including panniculus carnosus, and group II compound (II-1) has a promoting effect on healing of epidermis.

According to the above, it was found that the compound of the present invention promotes repair and regeneration selective to healing of complete skin-excised wound.

### (Formulation Example 1)

| Tablet | |
|---|---|
| (1) compound (I-1) | 10 g |
| (2) lactose | 90 g |
| (3) corn starch | 29 g |
| (4) magnesium stearate | 1 g |
| | 130 g |

The components (1) and (2) and 24 g of the component (3) were mixed with water to granulate. The resulting granules were mixed with 5 g of the component (3) and the component (4), and the mixture was compressed using a compressive tablettingmachine to prepare 1,000 tablets of 7 mm diameter containing 10 mg of the component (1) per tablet.

### (Formulation Example 2)

Tablets were similarly prepared as described above, except that compound (I-3), compound (II-1), compound (II-2), compound (III-1) and compound (III-2) were used respectively, instead of compound (I-1).

### (Formulation Example 3)

Tablets were similarly prepared as described above, except that 5 g of compound (I-1) and 5 g of compound (II-2), or 5 g of compound (I-3) and 5 g of compound (II-1) were used respectively, instead of 10 g of compound (I-1).

### (Formulation Example 4)

| Capsule | |
|---|---|
| (1) compound (I-1) | 50 mg |
| (2) lactose | 14 mg |
| (3) corn starch | 29 mg |
| (4) hydroxypropyl cellulose | 6 mg |
| (5) magnesium stearate | 1 mg |
| 100 mg per capsule | |

The components (1), (2), (3) and (4) were mixed and granulated according to a conventional method. The component (5) was added thereto and encapsulated into a gelatin capsule by a conventional method to give a capsule preparation.

### (Formulation Example 5)

Capsules were similarly prepared as described above, except that compound (I-3), compound (II-1), compound (II-2), compound (III-1) and compound (III-2) were used respectively, instead of compound (I-1).

### (Formulation Example 6)

Capsules were similarly prepared as described above, except that 5 g of compound (I-1) and 5 g of compound (II-2), or 5 g of compound (I-3) and 5 g of compound (II-1) were used respectively, instead of 10 g of compound (I-1).

### (Formulation Example 7)

| Ointment | |
|---|---|
| (1) compound (I-3) | 1 g |
| (2) compound (II-1) | 9 g |
| (3) white petrolatum | appropriate amount (total 100 g) |

The components (1), (2) and (3) were mixed to give an ointment.

### (Formulation Example 8)

### Emulsion

### (formulation)

(1) squalane 5.0% by weight, (2) white petrolatum 2.0% by weight, (3) bees wax 0.5% by weight, (4) sorbitan sesquioleate 0.8% by weight, (5) polyoxyethylene oleyl ether (20EO) 1.2% by weight, (6) methyl parahydroxy benzoate 0.1% by weight, (7) propylene glycol 5.0% by weight, (8) purified water to make a total amount of 100 % by weight, (9) 1.0% by weight solution of carboxy vinyl polymer in water 20.0% by weight, (10) potassium hydroxide 0.1% by weight, (11) ethanol 5.0% by weight, (12) flavorant 0.2% by weight, (13) sodium hyaluronate 0,3% by weight, (14) sodium salt of compound (I-4a) 0.5% by weight

### (preparation)

The oily components (1) to (5) were mixed and heated to 75°C to be dissolved uniformly. On the other hand, the aqueous components (6) to (8) were mixed, dissolved and heated to 75°C, and to this aqueous solution was added the above oily components to pre-emulsify. The component (9) was added to the pre-emulsion and then mixed with a homomixer to be emulsified uniformly, and the component (10) was added to adjust the pH. After cooling, the components (11) to (14) were added thereto at 40°C, and the mixture was homogenated.

### (Formulation Example 9)

Emulsions were similarly prepared as described above, except that 5 g of compound (I-3) and 5 g of compound (II-1), 5 g of compound (I-4a) and 5 g of compound (II-11a), or 3 g of compound (I-4a) and 7 g of compound (II-1) were used respectively, instead of 10 g of compound (I-4a).

### (Formulation Example 10)

### Lotion

### (formulation)

(1) ethanol 10.0% by weight, (2) 1,3-butylene glycol 5.0% by weight, (3) sodium salt of compound (II-11a) 0.1% by weight, (4) bovine placenta extract 0.1% by weight, (5) flavorant 0.1% by weight, (6) purified water 84.7% by weight

### (preparation)

(1) to (5) were added to (6) sequentially, and the mixture was mixed uniformly and dissolved.

### (Formulation Example 11)

### Skin cream

### (formulation)

(1) bees wax 6.0% by weight, (2) cetanol 5.0% by weight, (3) reduced lanolin 8.0% by weight, (4) squalane 27.5% by weight, (5) glyceryl fatty acid ester 4.0% by weight, (6) lipophilic glyceryl monostearic acid ester 2.0% by weight, (7) polyoxyethylene (20EO) sorbitan monolaurate 5.0% by weight, (8) propylene glycol 5.0% by weight, (9) methyl paraoxybenzoate 0.1% by weight, (10) purified water 36.7% by weight, (11) sodium salt of compound (I-4a) 0. 5% by weight, (12) lactic acid 0.2% by weight

### (preparation)

The oily components (1) to (7) were mixed, dissolved and heated to 75°C. On the other hand, the aqueous components (8) to (10) were mixed to be dissolved and heated to 75°C. Then, to the aqueous mixture was added the oily components to pre-emulsify, and then mixed with a homomixer to be emulsified uniformly. After cooling, (11) and (12) were added to the mixture at 40°C, and mixed.

### (Formulation Example 12)

### Milky foundation

### (formulation)

(1) stearic acid 2.0% by weight, (2) squalane 5.0% by weight, (3) octyldodecyl myristate 5.0% by weight, (4) cetanol 1.0% by weight, (5) decaglyceryl monoisopalmitate 9.0% by weight, (6) 1, 3-butylene glycol 6.0% by weight, (7) potassium hydroxide 0.1% by weight, (8) methyl paraoxybenzoate 0.1% by weight, (9) purified water 53.3% by weight, (10) titanium oxide 9.0% by weight, (11) talc 7.4% by weight, (12) red iron oxide 0.5% by weight, (13) yellow iron oxide 1.1% by weight, (14) black iron oxide 0.1% by weight, (15) flavorant 0.1% by weight, (16) sodium salt of compound(II-11a) 0.15% by weight, (17) 2-hydroxyacetic acid 0.15% by weight

### (preparation)

The oily components (1) to (5) were mixed and heated to 75°C to be uniform. On the other hand, the aqueous components (6) to (9) were mixed and heated to 75°C to be dissolved uniformly. The pigments (10) to (14) were added to the aqueous mixture and dispersed uniformly by using a homomixer. Then, to the aqueous mixture was added the oily components, and mixed with a homomixer to be emulsified uniformly. After cooling, (15) to (17) were added to the mixture at 40°C, and mixed.

### INDUSTRIAL APPLICABILITY

As regeneration-promoting CD11b⁺CD2⁺ macrophages and regeneration-promoting CD11b⁻CD2⁺ macrophages are lesion-selectively concerned with, for example, renal glomerular lesions and renal tubulointerstitial lesions, as well as epidermal lesions and dermal lesions, respectively, the pharmaceutical of the present invention containing the compound which promotes induction of these macrophages can be utilized as a preventive and therapeutic agent for renal glomerular lesions and renal tubulointerstitial lesions, as well as epidermal lesions and dermal lesions. In addition, concerning with effects on epidermal lesions and dermal lesions, a composition containing the compound which promotes induction of these macrophages can also be used as a cosmetic.

Further, each search for a compound initiating induction of regeneration-promoting CD11b⁺CD2⁺ macrophages and regeneration-promoting CD11b⁻CD2⁺ macrophages of the present invention can be applied for a screening method of each preventive and therapeutic agent lesion-selectively for, for example , renal glomerular lesions and renal tubulointerstitial lesions, as well as epidermal lesions and dermal lesions.

The present application is based on Japanese priority application Nos. 2002-265884 and 2003-159975 filed in Japan, and includes the entire contents thereof. References including patents and patent applications cited herein are hereby incorporated in equal level as the disclosed entire contents thereof by reference. Although the present invention has been described with respect to specific embodiments in the description and Examples, it is clear that the invention is not to be limited to the disclosed embodiments. In view of the foregoing description, various modifications and variations of the present invention may occur and are within the appended claims.

## Claims

1. A composition for prevention of sclerotic lesions causing apoptosis, degeneration, fibrosis and atrophy and/or for repair and regeneration of the aforementioned lesions, comprising a compound which preferentially increases regeneration-promoting macrophages.

2. A pharmaceutical for prevention and/or therapy of renal glomerular lesions, lesions of pancreatic islets of Langerhans or epidermal lesions , comprising a compound which preferentially increases regeneration-promoting CD11b⁺CD2⁺ macrophages.

3. The pharmaceutical as claimed in claim 2, wherein the compound which preferentially increasing regeneration-promoting CD11b⁺CD2⁺ macrophages is one or more compound(s) selected from the group consisting of (R)-1-naphthalen-2-ylethyl (R)-2-(4-fluorophenoxy)-5-oxotetrahydrofuran-2-carboxylate, 2-fluoro-5-oxotetrahydrofuran-2-carboxylic acid benzyl ester, bacitracin A, viomycin, 6,7-dimethoxy-1-morpholinomethyl-isochromane, 1-diethylaminomethyl-5-butoxy-6-methoxy-1,2,3,4-tetrahydroisoquinoline, 1-(4-fluorophenylthio)-2-methylaminopropanone, 2-chloro-5-oxotetrahydrofuran-2-carboxylic acid benzyl ester and 1-(2-oxo-hemiglutaric acid) benzyl ester.

4. A pharmaceutical for prevention and/or therapy of renal tubulointerstitial lesions, lesions of pancreatic exocrine or interstitial tissues, or dermal lesions, comprising a compound which promotes induction of human regeneration-promoting CD11b⁻CD2⁺ macrophages.

5. The pharmaceutical as claimed in claim 4, wherein the compound promoting induction of regeneration-promoting CD11b⁻CD2⁺ macrophages is one or more compound(s) selected from the group consisting of (R)-1-naphthalen-2-ylethyl (S)-2-(4-fluorophenoxy)-5-oxotetrahydrofuran-2-carboxylate, 2-benzyl-5-oxo-2-tetrahydrofuran carboxylic acid benzyl ester, 1-chloro-3-oxo-1,3-dihydroisobenzofuran-1-carboxylic acid benzyl ester and 1-(2-oxo-hemiglutaric acid) ethyl ester.

6. A pharmaceutical for prevention and/or therapy of kidney diseases, pancreatic diseases or skin diseases, comprising the compound described in claim 2 and the compound described in claim 4.

7. A pharmaceutical for prevention and/or therapy of kidney diseases, pancreatic diseases or skin diseases, comprising a compound which promotes induction of regeneration-promoting CD11b⁺CD2⁺ macrophages and regeneration-promoting CD11b⁻CD2⁺ macrophages.

8. The pharmaceutical as claimed in claim 7 , wherein the compound promoting induction of regeneration-promoting CD11b⁺CD2⁺ macrophages and regeneration-promoting CD11b⁻CD2⁺ macrophages is one or more compound(s) selected from the group consisting of 2-(4-chlorophenyl)thio-5-oxo-2-tetrahydrofuran carboxylic acid benzyl ester, 2-(4-fluorophenyl)oxy-5-oxo-2-tetrahydrofurancarboxylic acid ethyl ester, 2-(2,4-difluorophenyl)sulfonyl-5-oxo-2-tetrahydrofurancarboxylic acid benzyl ester, 2-phenoxy-5-oxo-2-tetrahydrofurancarboxylic acid benzhydryl ester, 2-(4-fluorophenyl)thio-5-oxo-2-tetrahydrofuran carboxylic acid, 2-(4-methoxyphenyl)thio-5-oxo-2-tetrahydrofurancarboxylic acid, 2-(2,4-difluorophenyl)thio-5-oxo-2-tetrahydrofurancarboxylic acid and 2-(4-fluorophenyl)sulfonyl-5-oxo-2-tetrahydrofurancarboxylic acid benzyl ester.

9. A cosmetic comprising a compound which promotes induction of regeneration-promoting CD11b⁺CD2⁺ macrophages.

10. The cosmetic as claimed in claim 9, wherein the compound promoting induction of regeneration-promoting CD11b⁺CD2⁺ macrophages is one or more compound(s) selected from the group consisting of (R)-1-naphthalen-2-ylethyl (R)-2-(4-fluorophenoxy)-5-oxotetrahydrofuran-2-carboxylate, 2-fluoro-5-oxotetrahydrofuran-2-carboxylic acid benzyl ester, bacitracin A, viomycin, 6,7-dimethoxy-1-morpholinomethyl-isochromane, 1-diethylaminomethyl-5-butoxy-6-methoxy-1,2,3,4-tetrahydroisoquinoline, 1-(4-fluorophenylthio)-2-methylaminopropanone, 2-chloro-5-oxotetrahydrofuran-2-carboxylic acid benzyl ester and 1-(2-oxo-hemiglutaric acid) benzyl ester.

11. A cosmetic comprising a compound which promotes induction of regeneration-promoting CD11b⁻CD2⁺ macrophages.

12. The cosmetic as claimed in claim 11, wherein the compound promoting induction of regeneration-promoting CD11b⁻CD2⁺ macrophages is one or more compound(s) selected from the group consisting of (R)-1-naphthalen-2-ylethyl (S)-2-(4-fluorophenoxy)-5-oxotetrahydrofuran-2-carboxylate, 2-benzyl-5-oxo-2-tetrahydrofurancarboxylic acid benzyl ester, 1-chloro-3-oxo-1,3-dihydroisobenzofuran-1-carboxylic acid benzyl ester and 1-(2-oxo-hemiglutaric acid) ethyl ester.

13. A cosmetic comprising the compound described in claim 9 and the compound described in claim 11.

14. A cosmetic comprising a compound which promotes induction of regeneration-promoting CD11b⁺CD2⁺ macrophages and regeneration-promoting CD11b⁻CD2⁺ macrophages.

15. The cosmetic as claimed in claim 14, wherein the compound promoting induction of regeneration-promoting CD11b⁺CD2⁺ macrophages and regeneration-promoting CD11b⁻CD2⁺ macrophages is one or more compound(s) selected from the group consisting of 2-(4-chlorophenyl)thio-5-oxo-2-tetrahydrofurancarboxylic acid benzyl ester, 2-(4-fluorophenyl)oxy-5-oxo-2-tetrahydrofurancarboxylic acid ethyl ester, 2-(2,4-difluorophenyl)sulfonyl-5-oxo-2-tetrahydrofurancarboxylic acid benzyl ester, 2-phenoxy-5-oxo-2-tetrahydrofurancarboxylic acid benzhydryl ester, 2-(4-fluorophenyl)thio-5-oxo-2-tetrahydrofuran carboxylic acid, 2-(4-methoxyphenyl)thio-5-oxo-2-tetrahydrofurancarboxylic acid, 2-(2,4-difluorophenyl)thio-5-oxo-2-tetrahydrofurancarboxylic acid and 2-(4-fluorophenyl)sulfonyl-5-oxo-2-tetrahydrofuran carboxylic acid benzyl ester.

16. A method for screening a compound which is able to prevent, mitigate or treat renal glomerular lesions, lesions of pancreatic islets of Langerhans or epidermal lesions, which comprises measuring a promoting action of a compound to be tested on the induction of regeneration-promoting CD11b⁺CD2⁺ macrophages and regulatory CD2⁻CD4⁺ T lymphocytes caused by contact of human peripheral blood mononuclear cells with a lipopolysaccharide.

17. A method for screening a compound which is able to prevent, mitigate or treat renal tubulointerstitial lesions, lesions of pancreatic exocrine or interstitial tissues, or dermal lesions, which comprises measuring a promoting action of a compound to be tested on the induction of regeneration-promoting CD11b⁻CD2⁺ macrophages and/or regulatory CD2⁻CD4⁺ T lymphocytes caused by contact of human peripheral blood mononuclear cells with mitomycin-treated human peripheral blood mononuclear cells.

18. A kit for screening a compound which is able to prevent, mitigate or treat renal glomerular lesions , lesions of pancreatic islets of Langerhans or epidermal lesions, which comprises (a) human peripheral blood mononuclear cells, (b) lipopolysaccharide and optionally (c) human AB type serum and/or (d) RPMI 1640 medium.

19. A kit for screening a compound which is able to prevent, mitigate or treat renal tubulointerstitial lesions, lesions of pancreatic exocrine or interstitial tissues, or dermal lesions, which comprises (a) human peripheral blood mononuclear cells, (b) mitomycin-treated human peripheral blood mononuclear cells and optionally (c) human AB type serum and/or (d) RPMI 1640 medium.

20. A kit for screening a compound which is able to prevent, mitigate or treat dermal lesions, which comprises (a) human peripheral blood mononuclear cells, (b) mitomycin-treated human peripheral blood mononuclear cells and optionally (c) human AB type serum and/or (d) RPMI 1640 medium.

21. A method for suppression and/or regeneration of sclerotic lesions causing apoptosis, degeneration, fibrosis and atrophy, which comprises administering a compound which preferentially increases regeneration-promoting macrophages to a patient.

22. A therapeutic method for renal glomerular lesions, lesions of pancreatic islets of Langerhans or epidermal lesions, which comprises administering a pharmaceutical comprising a compound which promotes induction of regeneration-promoting CD11b⁺CD2⁺ macrophages to a patient.

23. The therapeutic method as claimed in claim 22, wherein the compound which preferentially increases regeneration-promoting CD11b⁺CD2⁺ macrophages is one or more compound(s) selected from the group consisting of (R)-1-naphthalen-2-ylethyl (R)-2-(4-fluorophenoxy)-5- oxotetrahydrofuran-2-carboxylate, 2-fluoro-5-oxotetrahydrofuran-2-carboxylic acid benzyl ester, bacitracin A, viomycin, 6,7-dimethoxy-1-morpholinomethyl-isochromane, 1-diethylaminomethyl-5-butoxy-6-methoxy-1,2,3,4-tetrahydroisoquinoline, 1-(4-fluorophenylthio)-2-methylaminopropanone, 2-chloro-5-oxotetrahydrofuran-2-carboxylic acid benzyl ester and 1-(2-oxo-hemiglutaric acid) benzyl ester.

24. A therapeutic method for renal tubulointerstitial lesions, lesions of pancreatic exocrine or interstitial tissues , or dermal lesions, which comprises administering a pharmaceutical comprising a compound which promotes induction of regeneration-promoting CD11b⁻CD2⁺ macrophages to a patient.

25. The therapeutic method as claimed in claim 24, wherein the compound promoting induction of regeneration-promoting CD11b⁻CD2⁺ macrophages is one or more compound(s) selected from the group consisting of (R)-1-naphthalen-2-ylethyl (S)-2-(4-fluorophenoxy)-5-oxotetrahydrofuran-2-carboxylate, 2-benzl-5-oxo-2-tetrahydrofurancarboxylic acid benzyl ester, 1-chloro-3-oxo-1,3-dihydroisobenzofuran-1-carboxylic acid benzyl ester and 1-(2-oxo-hemiglutaric acid) ethyl ester.

26. A therapeutic method for kidney diseases, pancreatic diseases or skin diseases, which comprises concurrently administering the pharmaceutical described in claim 22 and the pharmaceutical described in claim 24 to a patient.

27. A therapeutic method for kidney diseases, pancreatic diseases or skin diseases, which comprises administering a compound which promotes induction of regeneration-promoting CD11b⁺CD2⁺ macrophages and regeneration-promoting CD11b⁻CD2⁺ macrophages to a patient.

28. The therapeutic method as claimed in claim 27, which comprises concurrently administering a compound promoting induction of regeneration-promoting CD11b⁺CD2⁺ macrophages and a compound promoting induction of regeneration-promoting CD11b⁻CD2⁺ macrophages, each of which being one or more compound(s) selected from the group consisting of 2-(4-chlorophenyl)thio-5-oxo-2-tetrahydrofuran carboxylic acid benzyl ester, 2-(4-fluorophenyl)oxy-5-oxo-2-tetrahydrofurancarboxylic acid ethyl ester, 2-(2,4-difluorophenyl)sulfonyl-5-oxo-2-tetrahydrofurancarboxylic acid benzyl ester, 2-phenoxy-5-oxo-2-tetrahydrofurancarboxylic acid benzhydryl ester, 2-(4-fluorophenyl)thio-5-oxo-2-tetrahydrofurancarboxylic acid, 2-(4-methoxyphenyl)thio-5-oxo-2-tetrahydrofurancarboxylic acid, 2-(2,4-difluorophenyl)thio-5-oxo-2-tetrahydrofurancarboxylic acid and 2-(4-fluorophenyl)sulfonyl-5-oxo-2-tetrahydrofurancarboxylic acid benzyl ester, to a patient.

29. Use of a compound which preferentially increases regeneration-promoting macrophages for production of a pharmaceutical which suppresses and/or regenerates sclerotic lesions causing apoptosis, degeneration, fibrosis and atrophy.

30. Use of a compound which promotes induction of regeneration-promoting CD11b⁺CD2⁺ macrophages for production of a pharmaceutical which prevents and/or treats renal glomerular lesions, lesions of pancreatic islets of Langerhans or epidermal lesions.

31. Use of a compound which promotes induction of regeneration-promoting CD11b⁺CD2⁺ macrophages for production of a cosmetic.

32. The use as claimed in claim 30 or claim 31, wherein the compound preferentially increasing regeneration-promoting CD11b⁺CD2⁺ macrophages is one or more compound(s) selected from the group consisting of (R)-1-naphthalen-2-ylethyl (R)-2-(4-fluorophenoxy)-5-oxotetrahydrofuran-2-carboxylate, 2-fluoro-5-oxotetrahydrofuran-2-carboxylic acid benzyl ester, bacitracin A, viomycin, 6,7-dimethoxy-1-morpholinomethyl-isochromane, 1-diethylaminomethyl-5-butoxy-6-methoxy-1,2,3,4-tetrahydroisoquinoline, 1-(4-fluorophenylthio)-2-methylaminopropanone, 2-chloro-5-oxotetrahydrofuran-2-carboxylic acid benzyl ester and 1-(2-oxo-hemiglutaric acid) benzyl ester.

33. Use of a compound which promotes induction of regeneration-promoting CD11b⁻CD2⁺ macrophages for production of a pharmaceutical which prevents or treats renal tubulointerstitial lesions, lesions of pancreatic exocrine or interstitial tissues, or dermal lesions.

34. Use of a compound which promotes induction of regeneration-promoting CD11b⁻CD2⁺ macrophages for production of a cosmetic.

35. The use as claimed in claim 33 or claim 34, wherein the compound promoting induction of regeneration-promoting CD11b⁻CD2⁺ macrophages is one or more compound(s) selected from the group consisting of (R)-1-naphthalen-2-ylethyl (S)-2-(4-fluorophenoxy)-5-oxotetrahydrofuran-2-carboxylate, 2-benzyl-5-oxo-2-tetrahydrofurancarboxylic acid benzyl ester, 1-chloro-3-oxo-1,3-dihydroisobenzofuran-1-carboxylic acid benzyl ester and 1-(2-oxo-hemiglutaric acid) ethyl ester.

36. Use of a compound which promotes induction of regeneration-promoting CD11b⁺CD2⁺ macrophages and regeneration-promoting CD11b⁻CD2⁺ macrophages for production of a pharmaceutical which prevents or treats kidney diseases, pancreatic diseases or skin diseases.

37. Use of a compound which promotes induction of regeneration-promoting CD11b⁺CD2⁺ macrophages and regeneration-promoting CD11b⁻CD2⁺ macrophages for production of a cosmetic.

38. The therapeutic method as claimed in claim 36 or claim 37, wherein the compound promoting induction of regeneration-promoting CD11b⁺CD2⁺ macrophages and regeneration-promoting CD11b⁻CD2⁺ macrophages is one or more compound(s) selected from the group consisting of 2-(4-chlorophenyl)thio-5-oxo-2-tetrahydrofurancarboxylic acid benzyl ester (III-1), 2-(4-fluorophenyl)oxy-5-oxo-2-tetrahydrofurancarboxylic acid ethyl ester (III-2), 2-(2,4-difluorophenyl)sulfonyl-5-oxo-2-tetrahydrofurancarboxylic acid benzyl ester, 2-phenoxy-5-oxo-2-tetrahydrofurancarboxylic acid benzhydryl ester, 2-(4-fluorophenyl)thio-5-oxo-2-tetrahydrofurancarboxylic acid, 2-(4-methoxyphenyl)thio-5-oxo-2-tetrahydrofurancarboxylic acid, 2-(2,4-difluorophenyl)thio-5-oxo-2-tetrahydrofurancarboxylic acid and 2-(4-fluorophenyl)sulfonyl-5-oxo-2-tetrahydrofurancarboxylic acid benzyl ester.
